(19)

Europäisches
Patentamt

European
Patent Office

Office européen
des brevets

(11) **EP 4 272 764 A1**

(12) **EUROPEAN PATENT APPLICATION**

(43) Date of publication:
**08.11.2023  Bulletin 2023/45**

(21) Application number: **22305661.5**

(22) Date of filing: **03.05.2022**

(51) International Patent Classification (IPC):
***A61K 47/69*** (2017.01)      ***C12N 5/00*** (2006.01)
***C12Q 1/6806*** (2018.01)

(52) Cooperative Patent Classification (CPC):
(C-Sets available)
**C12Q 1/6806; A61K 47/6901; G01N 33/48**   (Cont.)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicant: **Scipio Bioscience
92120 Montrouge (FR)**

(72) Inventors:
  • **ECKERT, Hélène
    92120 Montrouge (FR)**
  • **GRAINDORGE, Antoine Jacques
    92120 Montrouge (FR)**

  • **LE BOHEC, Maël Lois Franççois
    92120 Montrouge (FR)**
  • **BUSHKALOVA, Raya Dimchéva
    92120 Montrouge (FR)**
  • **KOMATSU, Jun
    92120 Montrouge (FR)**
  • **FERNANDEZ, Nicolas
    92120 Montrouge (FR)**

(74) Representative: **Icosa
    83 avenue Denfert-Rochereau
    75014 Paris (FR)**

Remarks:
The complete document including Reference
Table(s) and the Sequence Listing(s) can be
downloaded from the EPO website

(54) **METHOD OF COMPLEXING BIOLOGICAL UNITS WITH PARTICLES**

(57)    The invention relates to a method of complexing biological units with particles, by contacting biological units with particles in the presence of a thickening agent. The method can be implemented in a variety of applications, in particular in the field of single-cell analysis.

EP 4 272 764 A1

(52) Cooperative Patent Classification (CPC): (Cont.)

C-Sets
**C12Q 1/6806, C12Q 2527/153, C12Q 2535/122,**
**C12Q 2543/101, C12Q 2563/149,**
**C12Q 2563/179, C12Q 2565/519**

**Description**

**FIELD OF INVENTION**

The present invention relates to the field of single-cell analysis.

**BACKGROUND OF INVENTION**

Research in many areas of biology have benefited from studies at the single-cell level, particularly transcriptomics, with single-cell RNA sequencing (scRNA-seq) gaining in popularity mainly due to microfluidic-based methods, for which a strong early impetus was provided by key publications (Macosko et al., 2015. Cell. 161(5):1202-1214; Klein & Macosko, 2017. Lab Chip. 17(15):2540-2541). The full range of currently available methods has been presented in several reviews (Quake, 2021. Trends Genet. 37(12):1064-1068; Cable et al., 2021. Ann N Y Acad Sci. 1506(1):74-97), with the 10X Genomics Chromium instrument achieving particularly wide usage. Comparative benchmarking has been reported for many of the available methods (including CEL-seq2, MARS-seq, Quartz-seq2, gmcSCRB-seq, Smart-seq2, C1HT-small, C1HT-medium, Chromium, ddSEQ, Drop-seq, ICELL8, and inDrop) (Mereu et al., 2020. Nat Biotechnol. 38(6):747-755; Davis-Marcisak et al., 2021. Cancer Cell. 39(8):1062-1080).

Recent orientations of scRNA-seq have included major efforts to define atlases, notably for human and mouse cells, aided by technical optimization of methodologies. Other investigations have focused on specific organs, often in relation to disease states, especially cancer immunotherapy, as well as on development. Single-cell studies have also been extended to various forms of multiomics and methods that include spatial information.

In spite of the advances cited above, scRNA-seq methods do not always achieve sufficient transparency in preparation of samples for cDNA sequencing to permit users to evaluate performance at individual steps. As a result, unnecessary sequencing costs may be encountered for sample preparations that do not achieve minimal criteria. Considerations of processing time to avoid changes in transcriptome profiles and availability of resources must also be considered. Further optimization could include the following properties:

- instrument-free, so as to avoid an up-front investment for a limited number of trial experiments;
- independent of any requirement to reserve time an institute platform-based instrument, which many not coincide with the readiness of biological samples;
- sufficiently transparent to be able to monitor intermediate steps to assure quality control prior to sequencing;
- involving materials easily stored in individual laboratories and immediately available whenever required;
- following procedures with convenient stopping points to permit interruption of processing for later analysis of multiple samples in parallel or for clinical samples that arrive outside regular working hours;
- scalable for a wide range of numbers of cells in a sample, as well as cells of different sizes.

We have previously described a process that provided advances on the above points (WO 2018/203141).

Here, we went a step further and optimized this process to allow effective and reproducible results, in particular for complexing biological units (such as cells) with particles (such as barcode beads) in bulk solution in an efficient manner.

SUMMARY

The present invention relates to a method of complexing biological units with particles, comprising contacting a population of dissociated biological units with a solution comprising (i) a population of particles and (ii) at least one thickening agent, in the presence of at least one means for binding a biological unit, to obtain a suspension of biological unit-particle complexes, wherein the final viscosity of the suspension of biological unit-particle complexes ranges from about 3 mPa·s to about 15 mPa·s.

In some embodiments, the at least one means for binding a biological unit comprises a group or moiety selected from the group consisting of nucleic acids, lipids, proteins or fragments thereof, peptides, antibodies or fragments thereof, carbohydrates, vitamins or derivatives thereof, coenzymes or derivatives thereof, receptor ligands or derivatives thereof, and hydrophobic groups.

In some embodiments, the at least one means for binding a biological unit comprises at least a first nucleic acid molecule fused to a biological unit-binding moiety and at least a second nucleic acid molecule, wherein the at least second nucleic acid molecule is bound or otherwise attached to the particles of the population of particles, and wherein the at least first nucleic acid molecule and the at least second nucleic acid molecule are capable of hybridization.

In some embodiments, the method comprises the following steps in the recited order:

a) contacting the population of dissociated biological units with a biological unit-binding moiety fused to at least a first nucleic acid molecule, and

b) contacting the population of dissociated biological units from a) with the population of particles, wherein at least a second nucleic acid molecule is bound or otherwise attached to the particles of the population of particles;

wherein the at least first nucleic acid molecule and the at least second nucleic acid molecule are capable of hybridization.

The method according to claim **3** or **4,** wherein the biological unit-binding moiety is selected from the group consisting of lipids, antibodies or fragments thereof, and receptor ligands.

In some embodiments, the lipid is selected from the group consisting of sterol lipids, fatty acids, glycerolipids, glycerophospholipids, sphingolipids, saccharolipids, polyketides and prenol lipids; preferably wherein the lipid is cholesterol.

In some embodiments, the at least first nucleic acid molecule comprises or consists of a poly(dA) sequence, and the at least second nucleic acid molecule comprises or consists of a poly(dT) or poly(U) sequence.

In some embodiments, the biological units are biological structures comprising a lipid membrane, preferably selected from the group consisting of cells, nuclei, mitochondria, plastids, endoplasmic reticulum, Golgi apparatus, vacuoles, vesicles, organoids, spheroids, and cell tissues.

In some embodiments, the particles are beads.

In some embodiments, each particle in the population of particles comprises a unique identifier or clonal copies thereof, preferably the identifier is a nucleic acid barcode.

In some embodiments, the suspension of biological unit-particle complexes comprises:

- less than 50 % of uncomplexed biological units, and/or
- less than 10 % of biological units complexed with a particle concurrently with one or several other biological units.

The present invention also relates to a method of trapping discrete biological unit-particle complexes in a hydrogel, said method comprising the following steps:

a) complexing biological units with particles to form a suspension of biological unit-particle complexes according to the method of complexing biological units with particles described herein,

wherein the suspension of biological unit-particle complexes comprises, or is diluted with, a gellable solution optionally comprising at least one density-matching agent,

b) forming a hydrogel matrix trapping the biological unit-particle complexes, preferably in a discrete fashion.

The present invention also relates to a method of uniquely identifying a biological unit's analytes, said method comprising the following steps:

a) complexing biological units with particles to form a suspension of biological unit-particle complexes according to the method of complexing biological units with particles described herein,

wherein each particle comprises a unique identifier or clonal copies thereof, preferably the unique identifier is a nucleic acid barcode,

wherein the suspension of biological unit-particle complexes comprises, or is diluted with, a gellable solution optionally comprising at least one density-matching agent,

b) forming a hydrogel matrix trapping the biological unit-particle complexes, preferably in a discrete fashion, and

c) labelling the biological unit's analytes of each biological unit-particle complex with the unique identifier.

In some embodiments, the method comprises a further step of sequencing the biological unit's analytes labelled with their unique identifier.

The present invention also relates to a kit for implementing the methods described herein, comprising:

- a first container or vial containing at least a first nucleic acid molecule fused to a biological unit-binding moiety;
- a second container or vial containing a population of particles, said particles comprising at least a second nucleic acid molecule;
- at least one thickening agent, either in the second container or vial, or in a third container or vial,

wherein the at least first nucleic acid molecule and the at least second nucleic acid molecule are capable of hybridization.

**DEFINITIONS**

In this disclosure, any use of the singular forms **"a", "an"** or **"the"** includes the singular but also the plural references, unless the context clearly indicates otherwise. Thus, for example, a reference to "an agent" includes a single agent and a plurality of such agents.

**"About"** or **"approximately"** can mean within an acceptable error range for the particular value as determined by one of ordinary skill in the art, which will depend in part on how the value is measured or determined, *i.e.,* the limitations of the measurement system. For example, **"about"** can mean within 1 or more than 1 standard deviation, per the practice in the art. Alternatively, **"about"** preceding a figure means plus or less 10 % of the value of said figure. Alternatively, particularly with respect to biological systems or processes, the term can mean within an order of magnitude, within 5-fold, and more preferably within 2-fold, of a value. Where particular values are described in the application and claims, unless otherwise stated the term **"about"** meaning within an acceptable error range for the particular value should be assumed.

**"Amplification"** refers to the process of producing multiple copies, *i.e.,* at least 2 copies, of a desired template sequence. Techniques to amplify nucleic acids are well known to the skilled artisan, and include specific amplification methods as well as random amplification methods.

**"Barcode"** refers to a non-optical identifier. Barcodes typically comprise or consists of a biomolecular sequence, such as a nucleic acid or amino acid sequence. A barcode can be attached to an analyte in a reversible or irreversible manner. It can be added to, *e.g.,* a fragment of a DNA or RNA molecule before and/or during sequencing, so as to convey information about, *e.g.,* the source or origin of the DNA or RNA molecule.

**"Barcoding"** refers to the attachment of a barcode, such as a nucleic acid barcode, to a biological unit's analytes, in particular nucleic acid sequences, through primer template annealing, primer dependent DNA synthesis and/or ligation.

**"Bead"** refers to a particle that may be spherical (*e.g.,* microspheres) or have an irregular shape. Beads may be as small as about 0.1 $\mu$m in diameter or as large as about several millimeters in diameter.

**"Biological unit"** refers to a biological structure or a portion or component thereof, or to a combination of biological structures. Examples of biological units include, but are not limited to, cells, nuclei, mitochondria, plastids, endoplasmic reticulum, Golgi apparatus, vacuoles, vesicles, organoids, spheroids, and cell tissues, whether intracellular or extracellular, or combination thereof

**"cDNA library"** refers to a library composed of complementary DNAs which are reverse-transcribed from mRNAs.

**"Clonal copies"** refers to a population of identical copies of a barcode. Clonal copies may also be nearly identical, with sufficient identity, as the person skilled in the art will realize, to hybridize to identical sequences and/or achieve the same functions (*e.g.,* priming, identification, etc.). In preferred embodiments, two clonal copies share at least 70 % sequence identity, preferably at least 80 % sequence identity, preferably at least 90 % sequence identity, preferably at least 93 % sequence identity, and even more preferably at least 95 % sequence identity. In preferred embodiments, at least 60%, preferably at least 70%, preferably at least 80%, preferably at least 90% and even more preferably at least 95% of clonal copies are identical.

**"Coat"** and **"coating"** refer to the covering, modification or functionalization of a substrate, *e.g.,* of a particle such as a bead, or of a biological unit such as a cell.

**"Complement"** or **"complementary"** refer to a polynucleotide sequence capable of forming base-pairing by hydrogen bonds with another polynucleotide sequence, preferably Watson-Crick base-pairing unless explicitly specified otherwise. For example, guanine (G) is the Watson-Crick complementary base of cytosine (C), and adenine (A) is the Watson-Crick complementary base of thymine (T) and of uracil (U). Other types of base-pairing including, without limitation, Wobble base-pairing. For example, inosine (I) is a nucleoside which is capable of Wobble base-pairing with any natural base (C, T, U, A or G). Depending on the context, and as the person skilled in the art will clearly understand, "complementary" may mean "reverse complementary", *i.e.,* the sequences are complementary when read in opposite directions.

**"Hydrogel"** refers to a high water-content 3D network of hydrophilic polymers. These polymers in water are typically found in two states, depending among others on the extent of the interaction between individual polymer molecules: a solution (or liquid) state, and a solidified (or gel or polymerized) state. In the first state, the **"gellable solution"** behaves as a viscous liquid, while in the solidified state, the **"hydrogel"** exhibits a finite yield stress. The transition from the liquid state to the solidified state ("solidification") typically occurs in response to certain physical stimuli (such as resting time, changes in temperature, electric fields, magnetic fields, solvent composition, light intensity, and/or pressure) and/or chemical stimuli (such as changes in pH, ions, crosslinker addition, catalyst addition, enzyme activation, and/or specific chemical compositions).

Hydrogels can be classified into physical and chemical hydrogels based on their cross-linking mechanism. In one embodiment, hydrogels are prepared from at least one natural polymer. In one embodiment, hydrogels are prepared from at least one synthetic polymer. In one embodiment, hydrogels are prepared from at least one natural polymer and at least one synthetic polymer. Examples of physical hydrogel crosslinks include, but are not limited to, entangled chains, hydrogen bonding, hydrophobic interaction, ion capture, ion chelation and crystallite formation. Physical hydrogel can be synthesized by ionic interaction, crystallization, stereocomplex formation, hydrophobic interaction, protein interaction and hydrogen bond. Such physical hydrogels may be permanent or transient, reversible or irreversible. Examples of chemical hydrogels crosslinks include, but are not limited to, covalent bounds. Chemical hydrogels can be synthesized by chain growth polymerization, addition and condensation polymerization and gamma and electron beam polymerization. They can be formed by polymerization of end-functionalized macromers. Such chemical hydrogels can be permanent or transient, reversible or irreversible.

Some specific examples of hydrogels are polysaccharide hydrogels. These include, but are not limited to, alginate, agarose, κ-carrageenan, ι-carrageenan, chitosan, dextran, dialdehyde starch, heparin, gellan, native gellan gum, rhamsan, deacetylated rhamsan, S-657, xanthan gum and welan. Polysaccharide hydrogels can be formed by covalent crosslinking, ionic crosslinking, chemical conjugation, esterification and/or polymerization. By way of example, when the polysaccharide hydrogel is alginate, it can be crosslinked by ionic crosslinking in presence of a multivalent cation, such as calcium.

Some other specific examples of hydrogels are protein-based hydrogels. These include, but are not limited to, collagen, fibrin, albumin, gelatin, and laminin. Some protein-based hydrogels can be solidified by cooling or heating; they can also be solidified by crosslinking using a crosslinker, such as cyanamide, diisocyanate, dimethyl adipimidate, epoxy compounds, ethylaldehyde, formaldehyde, glutaraldehyde, glyceraldehyde, hexamethylenediamine, terephthalaldehyde and mixture thereof, optionally in the presence of a crosslinking activator, such as carbodiimide.

Some other specific examples of hydrogels are polysaccharide hydrogels combined with proteins as described here above. Hydrogels can also be non-biodegradable or/and synthetic hydrogels, including, but not limited to, compounds from vinylated monomers and/or vinylated macromers polymerization, in particular, 2-hydroxyethyl methacrylate, 2-hydroxypropyl methacrylate, acrylamide, acrylic acid, N-isopropylacrylamide, and poly N-isopropylacrylamide.

Some hydrogels, qualified as reversible hydrogels, may be able to transition back from their solidified state to a solution state: this phenomenon is classically referred to as "hydrogel depolymerization" or "hydrogel melting", which liquefaction can occur in response to certain chemical stimuli *(e.g.,* by changes in pH; changes of ionic concentrations [for instance, in the case of alginate]; by addition of reducing agent including, without limitation, phosphines [*e.g.,* tris(2-carboxyethyl)phosphine, *i.e.,* TCEP] or dithiothreitol, *i.e.,* DTT, such as in the case of albumin; by addition of oxidating agent; by addition of chelating agents, including, without limitation, EDTA and EGTA); by addition of organic solvent including, without limitation, ethanol and isopropanol; by addition of chaotropic compounds, including, without limitation, guanidine thiocyanate; by addition of kosmotropic compounds; by addition of a catalyst); or to certain thermal stimuli *(e.g.,* by temperature increase, such as in the case of thermosensitive or thermoreversible hydrogels if the temperature is raised above their melting point; by changes in electric field or magnetic field; by changes of pressure; by changes in light intensity; by mechanical stress, including, without limitation, vortexing, mixing, pipetting and grinding); or to certain enzymatic stimuli *(e.g.,* by enzymatic degradation, such as in the case of agarose using agarase). Hydrogels, once the gellable solution has solidified, exhibit diffusion properties that depend on the size of the diffusing objects. Small diffusing molecules diffuse like in a viscous media at rest while largest molecules interact with the 3D polymer network and diffuse slower. The typical size threshold between viscous-like diffusion and polymer-network interacting diffusion is usually called pore size and depends on numerous parameters such as polymer concentration, crosslinking point concentration, polymer persistence length and/or polymer hydration properties. In any of the embodiments described herein, the hydrogel should typically have a pore size sufficiently small to trap a biological unit, a barcode unit and/or an analyte extracted or derived from a biological unit *(i.e.,* to slow down their diffusion), while having a pore size sufficiently large to allow diffusion of biochemistry and molecular biology reagents. Such biochemistry and molecular biology reagents are well-known to the skilled artisan, and encompass all reagents known to perform biochemistry and molecular biology assays, such as solutions (buffer solutions, wash solutions, and the like), detergents, some enzymes, nucleic acid primers, and the like. These reagents can migrate in the hydrogel by diffusion, by convection or by the action of a field gradient *(e.g.,* by electrophoresis). Typical pore sizes for hydrogels range between about 1 nm and 1 μm, preferably from about 2 nm to about 500 nm, more preferably from about 5 nm to about 100 nm.

**"Identifier"** refers to a molecular pattern which can be used to uniquely identify a biological unit in a population of biological units. The term **"identifier"** further refers to the molecular pattern which conveys, or is capable of conveying, information about an analyte *(e.g.,* a nucleic acid such as a DNA molecule or fragment thereof, or an RNA such as an mRNA) and is therefore used to identify the source or origin of the analyte within a population of biological units, such as for example, a nucleic acid sequence extracted or derived from a biological unit in a population of biological units. Identifiers can be optical identifiers or non-optical identifiers. One example of identifier is a barcode.

**"Kit"** and **"kit-of-parts"** refer to any manufacture (*e.g.,* a package or at least one container) comprising the different reagents necessary for carrying out the methods described herein, packed so as to allow their transport and storage. The terms **"kit"** and **"kit-of-parts"** shall encompass an entity of physically separated components, which are intended for individual use, but in functional relation to each other. A kit may be promoted, distributed, or sold as a unit for performing the methods described herein. Furthermore, any or all of the kit reagents may be provided within containers that protect them from the external environment, such as in sealed and sterile containers. The kit may also contain a package insert describing the kit and methods for its use.

**"Lysis"** or **"lyse"** refer to the disruption of a biological unit in order to gain access to materials that are otherwise inaccessible. When the biological unit is a cell, lysis refers to breaking the cellular membrane of the cell, allowing transfer of reagents into the cell through cellular membrane holes and/or causing the cellular contents to spill out. Lysis methods are well-known to the skilled artisan, and include, but are not limited to, proteolytic lysis, chemical lysis, thermal lysis, mechanical lysis, and osmotic lysis.

**"Means for binding a biological unit"** refers to any molecule or group of molecules capable of binding, either covalently or non-covalently, to a biological unit. Non-limitative examples of means include nucleic acids, lipids, proteins or fragments thereof, peptides, antibodies or a antigen-binding fragments thereof, carbohydrates, vitamins or derivatives thereof, coenzymes or derivatives thereof, receptor ligands or derivatives thereof, and hydrophobic groups. Means for binding a biological unit may comprise a single molecule, *e.g.,* a molecule attached or otherwise bound to a particle as described herein, and interacting directly with a biological unit. Alternatively, means for binding a biological unit may comprise a group of molecules interacting together to form a bridge between a particle and a biological unit, wherein at least one molecule of the group of molecules is attached or otherwise bound to a particle as described herein.

**"Nucleic acid sequence primer",** or simply **"primer",** refers to an oligonucleotide that is capable of hybridizing or annealing with a nucleic acid and serving as an initiation site for nucleotide polymerization under appropriate conditions, such as the presence of nucleoside triphosphates and an enzyme for polymerization, such as DNA or RNA polymerase or reverse transcriptase, in an appropriate buffer and at a suitable temperature.

**"Oligonucleotide"** refers to a polymer of nucleotides, generally to a single-stranded polymer of nucleotides. In some embodiments, the oligonucleotide comprises from 2 to 500 nucleotides, preferably from 10 to 150 nucleotides, preferably from 20 to 100 nucleotides. Oligonucleotides may be synthetic or may be made enzymatically. In some embodiments, oligonucleotides may comprise ribonucleotide monomers, deoxyribonucleotide monomers, or a mix of both. As the skilled person will realize, nucleotide analogs (such as peptide nucleic acids, locked nucleic acids, glycol nucleic acids, etc.) may replace some or all of the nucleotides in an oligonucleotide.

**"Polymerase chain reaction",** abbreviated as **"PCR",** refers to methods including, but not limited to, allele-specific PCR, asymmetric PCR, hot-start PCR, intersequence-specific PCR, methylation-specific PCR, miniprimer PCR, multiplex ligation-dependent probe amplification, multiplex-PCR, nested PCR1 quantitative PCR, reverse transcription PCR and/or touchdown PCR. DNA polymerase enzymes suitable to amplify nucleic acids comprise, but are not limited to, Taq polymerase Stoffel fragment, Taq polymerase, Advantage DNA polymerase, AmpliTaq, AmpliTaq Gold, Titanium Taq polymerase, KlenTaq DNA polymerase, Platinum Taq polymerase, Accuprime Taq polymerase, Pfu polymerase, Pfu polymerase turbo, Vent polymerase, Vent exo- polymerase, Pwo polymerase, 9 Nm DNA polymerase, Therminator, Pfx DNA polymerase, Expand DNA polymerase, rTth DNA polymerase, DyNAzyme-EXT Polymerase, Klenow fragment, DNA polymerase I, T7 polymerase, SequenaseTM, Tfi polymerase, T4 DNA polymerase, Bst polymerase, Bca polymerase, BSU polymerase, phi-29 DNA polymerase and DNA polymerase Beta or modified versions thereof. In one embodiment, the DNA polymerase has a 3'-5' proofreading, *i.e.,* exonuclease, activity. In one embodiment, the DNA polymerase has a 5'-3' proofreading, *i.e.,* exonuclease, activity. In one embodiment, the DNA polymerase has strand displacement activity, *i.e.,* the DNA polymerase causes the dissociation of a paired nucleic acid from its complementary strand in a direction from 5' towards 3', in conjunction with, and close to, the template-dependent nucleic acid synthesis. DNA polymerases such as *E. coli* DNA polymerase I, Klenow fragment of DNA polymerase I, T7 or T5 bacteriophage DNA polymerase, and HIV virus reverse transcriptase are enzymes which possess both the polymerase activity and the strand displacement activity. Agents such as helicases can be used in conjunction with inducing agents which do not possess strand displacement activity in order to produce the strand displacement effect, that is to say the displacement of a nucleic acid coupled to the synthesis of a nucleic acid of the same sequence. Likewise, proteins such as Rec A or Single-Strand DNA Binding Protein (SSB) from *E. coli* or from another organism could be used to produce or to promote the strand displacement, in conjunction with other inducing agents (Kornberg A. & Baker T.A. (1992). Chapters 4-6. In DNA replication (2nd Ed., pp. 113-225). New York: W.H. Freeman).

"Primer-directed extension" refers to any method known in the art wherein primers are used to initiate replication of nucleic acid sequences in the linear or logarithmic amplification of nucleic acid molecules. Primer-directed extension may be accomplished by any of several schemes known in this art including, but not limited to, polymerase chain reaction (PCR), ligase chain reaction (LCR) and strand-displacement amplification (SDA). **"Primer-directed extension"** can be carried out by DNA polymerase enzymes as described herein.

**"Random amplification techniques"** include, without limitation, multiple displacement amplification (MDA), random PCR, random amplification of polymorphic DNA (RAPD) or multiple annealing and looping based amplification cycles (MALBAC). By opposition, **"specific amplification techniques"** include without limitation, methods requiring temperature cycling (such as polymerase chain reaction (PCR), ligase chain reaction, transcription-based amplification) and/or isothermal amplification systems (such as self-sustaining sequence replication, replicase system, helicase system, strand displacement amplification, rolling circle-based amplification and NASBA).

**"Reverse transcription"** refers to the replication of RNA to produce DNA complementary strands DNA (cDNA), in particular using an RNA-directed DNA polymerase (such as, *e.g.*, reverse transcriptase, RT). The reverse-transcription of RNAs may be carried out by techniques well known to the skilled artisan, using a reverse transcriptase enzyme and a mix of 4 deoxyribonucleotides triphosphate (dNTPs), namely deoxyadenosine triphosphate (dATP), deoxycytidine triphosphate (dCTP), deoxyguanosine triphosphate (dGTP) and (deoxy)thymidine triphosphate (dTTP). In some embodiments, the reverse-transcription of RNAs comprises a first step of first-strand cDNA synthesis. Methods for first-strand cDNA synthesis are well-known to the skilled artisan. First-strand cDNA synthesis reactions can use a combination of sequence-specific primers, oligo(dT) primers or random primers. Examples of reverse transcripatase enzymes include, but are not limited to, M-MLV reverse transcriptase, SuperScript II (Invitrogen), SuperScript III (Invitrogen), SuperScript IV (Invitrogen), Maxima (ThermoFisher Scientific), ProtoScript II (New England Biolabs), PrimeScript (ClonTech). In some embodiments, the reverse transcription can be a primer-directed extension. In these embodiments, the reverse transcription requires a single-stranded DNA molecule at least comprising a probing sequence capable of hybridization with a portion of the RNA and serving as amplification primer site. The single-stranded DNA molecule may further contain other functional sequences. In some embodiments, the single-stranded DNA molecule is an oligonucleotide bound or otherwise attached to a particle as will be described herein, comprising *inter alia* a unique identifier such as a nucleic acid barcode. cDNA polymerization starts from the amplification primer site, and the resulting cDNA molecule (so-called "first-strand cDNA molecule") contains (i) the original single-stranded DNA molecule including the functional sequences when present and (ii) the complementary DNA sequence of the hybridized RNA.

**"Second-strand synthesis"** refers to the synthesis of a complementary sequence to the first-strand cDNA molecule to produce an at least partially double-stranded DNA molecule. Second-strand synthesis can be implemented to obtain cDNA replicates in solution without the need for an intermediate step such as an extraction step *(e.g.,* in case of chemical contamination) or molecular cleavage *(e.g.,* in case of a particle-grafted cDNA). In some embodiments, second-strain synthesis can be a primer-directed extension. In these embodiments, the second-strand synthesis requires a single-stranded DNA molecule at least comprising a probing sequence, at its 3'-end,apable of hybridization with a portion of the first-strand cDNA molecule and serving as amplification primer site. In some embodiments, these primers are random oligonucleotides. In some embodiments, the primers are specific to some target sequences. The single-stranded DNA molecule may further contain other functional sequences located in 5'-direction from the probing sequence, such as, *e.g.,* unique molecular identifier (UMI), PCR handle and/or nucleic acid barcode. In these embodiments, second-strand synthesis starts from the amplification primer site and the resulting DNA molecule (so-called "second-strand cDNA molecule") contains (i) the original single-stranded DNA molecule including the functional sequences when present and (ii) the complementary DNA sequence of the first-strand cDNA molecule. Methods for second-strand cDNA synthesis are well-known to the skilled artisan, and can be implemented using DNA-dependent DNA polymerases, such as, *e.g.,* Taq polymerase Stoffel fragment, Taq polymerase, Advantage DNA polymerase, AmpliTaq, AmpliTaq Gold, Titanium Taq polymerase, KlenTaq DNA polymerase, Platinum Taq polymerase, Accuprime Taq polymerase, Pfu polymerase, Pfu polymerase turbo, Vent polymerase, Vent exo- polymerase, Pwo polymerase, 9 Nm DNA polymerase, Therminator, Pfx DNA polymerase, Expand DNA polymerase, rTth DNA polymerase, DyNAzyme-EXT Polymerase, Klenow fragment, DNA polymerase I, T7 polymerase, SequenaseTM, Tfi polymerase, T4 DNA polymerase, Bst polymerase, Bca polymerase, BSU polymerase, phi-29 DNA polymerase and DNA polymerase Beta or modified versions thereof. In some embodiments, the DNA-dependent DNA polymerase has a 3'-5' proofreading activity. In some embodiments, the DNA-dependent DNA polymerase has a 5'-3' proofreading activity. In some embodiments, the DNA-dependent DNA polymerase has a 5'-3' exonuclease activity. In some embodiments, the DNA-dependent DNA polymerase has strand displacement activity, *i.e.*, the DNA-dependent DNA polymerase causes the dissociation of a paired nucleic acid from its complementary strand in a direction from 5' towards 3', in conjunction with, and close to, the template-dependent nucleic acid synthesis. DNA-dependent DNA polymerase such as *E. coli* DNA polymerase I, Klenow fragment of DNA polymerase I, T7 or T5 bacteriophage DNA polymerase, and HIV reverse transcriptase are enzymes which possess both a polymerase activity and a strand displacement activity.

**"Particle"** refers to a particulate solid or semi-solid support. It may be rigid, flexible and/or compressible. It may be disruptable or dissolvable. It may be magnetic or nonmagnetic. A non-limiting example of particle is a bead.

**"Template"** or **"template sequence"** refer to a nucleic acid sequence for which amplification is desired. A template can comprise DNA or RNA. The template sequence may be known or unknown.

**"Thickening agent"** refers to a substance which can increase the apparent viscosity of a liquid without substantially changing its other properties. It can thus be referred to as **"viscosity-increasing agent".**

**"Viscosity"** refers to the measure of a fluid's resistance to irreversible deformation at a given rate. Viscosity is sometimes informally referred to as "thickness". Viscosity can be measured using apparatus known as viscometers and rheometers. For instance, a rheometer can be used for fluids that cannot be defined by a single value of viscosity and therefore require more parameters to be set and measured than is the case for a viscometer. Temperature control of the fluid is essential to obtain accurate measurements. Methods for measuring the viscosity of a fluid are described in the Example section. Viscosity of a fluid can be expressed in newton-second per square meter ($N \cdot s/m^2$), in pascal-second ($Pa \cdot s$), kilogram per meter per second ($kg \cdot m^{-1} \cdot s^{-1}$), Poiseuilli (PI) or poise (P, or $g \cdot cm^{-1} \cdot s^{-1}$).

## DETAILED DESCRIPTION

The present invention relates to a method of complexing biological units with particles, comprising contacting a population of dissociated biological units with a solution comprising (i) a population of particles and (ii) at least one thickening agent, in the presence of at least one means for binding a biological unit, to obtain a suspension of biological unit-particle complexes,
wherein the final viscosity of the suspension of biological unit-particle complexes ranges from about 3 mPa·s to about 15 mPa·s.

By **"complexing"**, it is meant coupling one or several biological units with one or several particles, preferably one biological unit with one particle, to form a biological unit-particle complex. Preferably, the biological unit-particle complex is stable under standard conditions, such as at room temperature or at 37°C, at physiological pH, meaning that the biological unit-particle complex does not dissociate under these conditions. Complexation (or coupling) occurs *inter alia* via the at least one means for binding a biological unit.

By **"contacting a population of dissociated biological units with a solution comprising (i) a population of particles and (ii) at least one thickening agent"**, it is meant any action that results in the population of dissociated biological units being in the same volume together with the solution comprising the population of particles and the at least one thickening agent. Alternatively, it is meant any action that results in at least one biological unit of the population of dissociated biological units physically contacting at least one particle of the population of particles.

In some embodiment, contacting the population of dissociated biological units with the solution comprising the population of particles and the at least one thickening agent comprises exposing the population of dissociated biological units to the solution.

The solution comprising the population of particles and the at least one thickening agent may be added to the population of dissociated biological units. Alternatively, the population of dissociated biological units may be added to the solution comprising the population of particles and the at least one thickening agent.

In some embodiments, contacting the population of dissociated biological units with the solution comprising the population of particles and the at least one thickening agent further comprises mixing the population of dissociated biological units and the solution comprising the population of particles and the at least one thickening agent together.

The skilled artisan is aware of means and method for mixing two components together. These include, without limitation, pipetting up and down; using a magnetic stirrer, a tube rotator, a rocking platform, etc.; manual shaking; etc.

Any means for mixing the population of dissociated biological units and the solution comprising the population of particles and the at least one thickening agent together may be suitable, as long as it creates and/or maintains a relative motion between the biological units and the particles, preferably as long as it creates and/or maintains a turbulent environment, thereby increasing the odds of having a biological unit physically entering into contact with a particle.

The population of biological units may comprise at least about 10, 100, 500, 1 000, 2 000, 5 000, 10 000, 20 000, 50 000, 100 000, 1 000 000, 5 000 000, 10 000 000 or more biological units. In preferred embodiments, the population of biological units comprises from 100 to 100 000 biological units, from 500 to 50 000 biological units, from 500 to 20 000 biological units or from 2 000 to 10 000 biological units.

In preferred embodiments, the concentration of biological units in the step of complexing biological units with particles is no lower than 0.01, 0.1, 1, 5, 10, or 50 biological units per µL and/or no higher than 1 000, 500, 250, or 100 biological units per µL. In preferred embodiments, the concentration of biological units in the step of complexing biological units with particles ranges from 1 to 1 0 00 biological units per µL, preferably from 5 to 500 biological units per µL, more preferably from 10 to 250 biological units per µL, or even more preferably from 50 to 100 biological units per µL.

The biological units may be any biological structure or portion or component thereof. In particular, the biological units may be any biological structure or portion or component thereof, comprising a lipid membrane, preferably a lipid bilayer.

In some embodiments, the biological units are selected from the group consisting of cells, nuclei, mitochondria, plastids, endoplasmic reticulum, Golgi apparatus, vacuoles, vesicles, organoids, spheroids, and cell tissues.

In some embodiments, the biological units are cells. Examples of cells include, but are not limited to, cells in *in vitro* culture; stem cells such as embryonic stem cells, adult stem cells, cancer stem cells, induced pluripotent stem cells or induced stem cells; tumor cells; tissue biopsy cells; blood cells such as erythrocytes, leukocytes, mast cells, macrophages, thrombocytes or progenitor cells thereof; tissue section cells; plant cells; and unicellular organisms.

The biological units are preferably dissociated, *i.e.*, the biological units are separated into single (or individual) biological units. Preferably, a majority, *i.e.,* more than 50 % of the biological units are dissociated, preferably more than 60 %, 70 %, 80 %, 90 % or more of the biological units are dissociated. For instance, when the biological unit is a cell, a tissue can be dissociated into individual cells by methods well-known in the art, including by enzymatic dissociation, chemical dissociation or mechanical dissociation. For instance, when the biological unit is a cell tissue, a tissue can be dissociated into individual groups of cells, such as groups of cells sharing a same function and/or structure. Enzymatic dissociation is a process of using enzymes to digest tissue pieces, thereby releasing individual cells (or groups of cells) from the tissue. Many different types of enzymes can be used in this process. Chemical dissociation takes advantage of the fact that cations participate in the maintenance of intracellular bonds and the intracellular matrix. By introducing EDTA or EGTA, which binds these cations, the intercellular bonds can be disrupted. Mechanical dissociation requires cutting, scraping or scratching a tissue into small pieces, then washing the minced-up tissue in order to separate the cells from the tissue.

According to the invention, the population of biological units is contacted with a solution comprising a (i) a population of particles and (ii) at least one thickening agent.

The population of particles may comprise at least about 1 000, 5 000, 10 000, 50 000, 100 000, 1 000 000, 5 000 000, 10 000 000 or more particles.

In preferred embodiments, the population of particles in the step of complexing biological units with particles comprises at least 1 time the number of biological units, preferably at least 2 times the number of biological units, preferably at least 5 times the number of biological units and even more preferably at least 8 times the number of biological units.

In preferred embodiments, the population of particles in the step of complexing biological units with particles comprises no more than 100 times the number of biological units, preferably no more than 50 times the number of biological units, 25 times the number of biological units, and even more preferably no more than 15 times the number of biological units.

In preferred embodiments, the population of particles in the step of complexing biological units with particles comprises between 2 times the number of biological units and 50 times the number of biological units, preferably between 5 times the number of biological units and 20 times the number of biological units, and even more preferably between 8 times the number of biological units and 15 times the number of biological units.

In preferred embodiments, the population of particles in the step of complexing biological units with particles comprises about 10 times the number of biological units.

The particles may be solid or semi-solid particles.

Particles may be made in any material, including, but not limited to, acrylics, carbon (*e.g.,* graphite, carbon-fiber), cellulose (*e.g.,* cellulose acetate), ceramics, controlled-pore glass, cross-linked polysaccharides (*e.g.,* agarose, SEPHAROSE™ or alginate), gels, glass (*e.g.,* modified or functionalized glass), gold (*e.g.,* atomically smooth Au(111)), graphite, inorganic glasses, inorganic polymers, latex, metal oxides (*e.g.,* $SiO_2$, $TiO_2$, stainless steel), metalloids, metals (*e.g.,* atomically smooth Au(111)), mica, molybdenum sulfides, nanomaterials (*e.g.,* highly oriented pyrolitic graphite (HOPG) nanosheets), nitrocellulose, NYLON™, optical fiber bundles, organic polymers, paper, plastics, polacryloylmorpholide, poly(4-methylbutene), polyethylene terephthalate), poly(vinyl butyrate), polybutylene, polydimethylsiloxane (PDMS), polyethylene, polyformaldehyde, polymethacrylate, polypropylene, polysaccharides, polystyrene, polyurethanes, polyvinylidene difluoride (PVDF), quartz, rayon, resins, rubbers, semiconductor material, silica, silicon (*e.g.,* surface-oxidized silicon), sulfide, TEFLON™, and combinations thereof.

The particles may have any shape, but are preferably 3-dimentional particles. In some embodiments, the particles have a general spherical shape.

In some embodiments, the particles have a mean diameter ranging from about 1 μm to about 500 μm, preferably from about 1 μm to about 250 μm, from about 1 μm to about 100 μm, from about 1 μm to about 50 μm. In some embodiments, the particles have a mean diameter of about 1 μm, 2 μm, 3 μm, 4 μm, 5 μm, 6 μm, 7 μm, 8 μm, 9 μm, 10 μm, 11 μm, 12 μm, 13 μm, 14 μm, 15 μm, 16 μm, 17 μm, 18 μm, 19 μm, 20 μm, 21 μm, 22 μm, 23 μm, 24 μm, 25 μm, 26 μm, 27 μm, 28 μm, 29 μm, 30 μm, 31 μm, 32 μm, 33 μm, 34 μm, 35 μm, 36 μm, 37 μm, 38 μm, 39 μm, 40 μm, 41 μm, 42 μm, 43 μm, 44 μm, 45 μm, 46 μm, 47 μm, 48 μm, 49 μm, 50 μm.

In some embodiments, the particles are beads.

In some embodiments, each particle in the population of particles comprises a unique identifier (*i.e.,* an identifier which is different from one particle to another in the population of particles). In some embodiments, each particle in the population of particles comprises clonal copies of a unique identifier.

It may indeed be desirable to uniquely identify a biological unit in a population of biological units, or its analytes (*e.g.,* its genome [a DNA molecule or fragment thereof] or transcriptome [an RNA molecule such as an mRNA]), in particular when the analytes of several biological units have been mixed and are interspersed or solubilized in a common liquid. This ability to attribute characteristics to individual biological units or analytes thereof is provided by the assignment of a unique identifier specifically to an individual biological unit or its analytes.

Identifiers may be of a variety of different formats, including labels, tags, probes, and the like.

The identifier may be an optical identifier, or a non-optical identifier.

Optical identifiers include, but are not limited to, chromophores, fluorophores, quantum dots, styrene monomers, and combination thereof, which can be identified, *e.g.,* by their spectrum such as Raman spectrum or electromagnetic spectrum; and/or by their intensity, color and the like.

Non-optical barcodes include, but are not limited to, biomolecular sequences such as DNA, RNA and/or protein sequences, which can be identified, *e.g.,* by sequencing techniques.

When a particle comprises clonal copies of a unique identifier, the number of copies can range from about 2 to about $10^{12}$.

In some embodiments, the identifier is preferably a non-optical identifier, in particular a nucleic acid barcode.

A nucleic acid barcode may be single-stranded or double-stranded, preferably single-stranded. It can be a DNA barcode, an RNA barcode, or a mix of both.

A nucleic acid barcode can include from 5 to 20 or more nucleotides. In some cases, the length of a nucleic acid barcode may be 5, 6, 7, 8, 9, 10, 11, 12, 13, 14, 15, 16, 17, 18, 19, 20 nucleotides. In some cases, the length of a nucleic acid barcode may be at least 5, 6, 7, 8, 9, 10, 11, 12, 13, 14, 15, 16, 17, 18, 19, 20 nucleotides or longer. In some cases, the length of a nucleic acid barcode may be at most 5, 6, 7, 8, 9, 10, 11, 12, 13, 14, 15, 16, 17, 18, 19, 20 nucleotides or shorter. In preferred embodiments, the length of a nucleic acid barcode may be 5 to 20 nucleotides, preferably 8 to 16 nucleotides, 9 to 16 nucleotides and even more preferably 10 to 14 nucleotides. These nucleotides may be contiguous, *i.e.,* in a single stretch of adjacent nucleotides, or they may be separated into two or more separate subsequences that are separated by 1 or more nucleotides.

In some embodiments, all the nucleic acid barcodes attached to a particular particle include the same nucleic acid barcode sequence (*i.e.,* clonal copies of a nucleic acid barcode), but each particle in a population of particles, or most particles in a population of particles, comprise a different nucleic acid barcode sequence, such that a large number of diverse nucleic acid barcode sequences are represented across a population of particles, *e.g.,* at least about 1 000, 5 000, 10 000, 50 000, 100 000, 1 000 000, 5 000 000, 10 000 000, 50 000 000, 100 000 000, 500 000 000, 1 000 000 000 or more different nucleic acid barcode sequences.

In some embodiments, each particle in the population of particles comprises at least one oligonucleotide, said oligonucleotide comprising (i) a nucleic acid barcode as detailed above and (ii) at least one other functional nucleic acid sequence.

These other functional nucleic acid sequences may be useful for processing the analytes, in particular the nucleic acids, from the biological units. Such processing may include immobilization, replication, extension and/or amplification of analytes, in particular of nucleic acids.

These functional nucleic acid sequences include, for example, targeted or random/universal amplification primer sequences, sequencing primers or primer recognition sites, hybridization or probing sequences, or any of a number of other potential functional sequences.

In some embodiments, the oligonucleotide comprises a hybridization or probing sequence.

The hybridization or probing sequence may be single-stranded or double-stranded, preferably single-stranded. It may be degenerate (*i.e.,* with a random sequence) or be specific of a nucleic acid sequence of interest.

The hybridization or probing sequence may in particular comprise or consist of a poly(dT) sequence or a poly(U) sequence. Accordingly, the hybridization or probing sequence is specific of a poly(dA) sequence. Poly(dA) sequences may be found, *e.g.,* at the 3'end of mRNAs, within the poly-A tail.

The hybridization or probing sequence may in particular comprise or consist of the sequence $(dT)_nVN$ or $(U)_nVN$ (from 5' to 3'), wherein n ranges from 5 to 50, V represents any nucleotide but dT/U (*i.e.,* dA, dC or dG), and N represents any nucleotide (*i.e.,* dA, dT, U, dC or dG). Accordingly, the hybridization or probing sequence is specific of a $NB(A)_n$ sequence (from 5' to 3'), wherein n ranges from 5 to 50, B represents any nucleotide but dA (*i.e.,* dT, U, dC or dG), and N represents any nucleotide (*i.e.,* dA, dT, U, dC or dG). $NB(A)_n$ sequences may be found, *e.g.,* at the 3'end of mRNAs, overlapping between the poly-A tail and the 3'UTR or CDS.

The hybridization or probing sequence may in particular comprise or consist of a poly-inosine (poly(I)) sequence. Inosine is a nucleoside which is capable of Wobble base-pairing with any natural base. Accordingly, the hybridization or probing sequence is non-specific and can prime to any nucleic acid sequence.

In some embodiments, the hybridization or probing sequence is identical in every oligonucleotide of a given particle; preferably the hybridization or probing sequence is identical in every oligonucleotide of every particle of the population of particles. In some embodiments, the hybridization or probing sequence of each oligonucleotide of a given particle is one among a set of probing sequences, such set of probing sequences comprising at least 2 and at most 10 000 distinct probing sequences, or at least 2 and at most 1000 distinct probing sequences. In preferred such embodiments, the set of probing sequences comprises at least 2, at least 5, at least 10, at least 20, at least 50, or at least 100 distinct probing sequences, and at most 10 000, at most 5 000, at most 1 000, or at most 500 probing sequences. Such set of probing sequences allows, *inter alia,* for each given particle to capture different sets of nucleic acid sequences from a given biological unit.

In some embodiments, the hybridization or probing sequence comprises from 5 to 50 or more nucleotides. In some cases, the length of the hybridization or probing sequence may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides. In some cases, the length of the hybridization or probing sequence may be at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides or longer. In some cases, the length of the hybridization or probing sequence may be at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50 nucleotides or shorter.

In preferred embodiments, the length of the hybridization or probing sequences may range from 5 to 100, preferably from 10 to 50, even more preferably from 20 to 40 nucleotides.

In some embodiments, the oligonucleotide comprises a PCR handle sequence.

By **"PCR handle sequence",** it is meant a nucleic acid sequence useful for enabling amplification, preferably PCR amplification and further sequencing of nucleic acid sequences extracted or derived from a biological unit.

In some embodiments, the PCR handle sequence is identical in every oligonucleotide of a given particle; preferably the PCR handle sequence is identical in every oligonucleotide of every particle of the population of particles.

In some embodiments, the PCR handle sequence comprises from 10 to 30 or more nucleotides. In some cases, the length of the PCR handle sequence may be 10, 11, 12, 13, 14, 15, 16, 17, 18, 19, 20, 21, 22, 23, 24, 25, 26, 27, 28, 29, 30 nucleotides. In some cases, the length of the PCR handle sequence may be at least 10, 11, 12, 13, 14, 15, 16, 17, 18, 19, 20, 21, 22, 23, 24, 25, 26, 27, 28, 29, 30, 31, 32, 33, 34, 34, 35, 36, 37, 38, 39, 40, 41, 42, 43, 44, 45, 46, 47, 48, 49, 50 nucleotides or longer. In some cases, the length of the PCR handle sequence may be at most 10, 11, 12, 13, 14, 15, 16, 17, 18, 19, 20, 21, 22, 23, 24, 25, 26, 27, 28, 29, 30, 31, 32, 33, 34, 34, 35, 36, 37, 38, 39, 40, 41, 42, 43, 44, 45, 46, 47, 48, 49, 50 nucleotides or shorter.

In preferred embodiments, the length of the PCR handle ranges from 15 to 35 nucleotides, preferably from 20 to 30 nucleotides, more preferably the length of the PCR handle is 25 nucleotides.

In some embodiments, the oligonucleotide comprises a unique molecular identifier (UMI).

By **"unique molecular identifier"** or **"UMI"**, it is meant a nucleic acid sequence serving as an index, useful for reducing errors and quantitative bias introduced by amplification steps.

In some embodiments, the unique molecular identifier is different in each oligonucleotide of a given particle. As the person skilled in the art will realize, it is not necessary for the UMI in each oligonucleotide of a given particle to be different to allow unambiguous identification of amplified molecules, as molecules may be distinguished by the combination of the UMI and of the amplified biological unit's analyte (e.g., the mRNA sequence). In some embodiments, the oligonucleotides of a given particle comprise a wide variety of different UMIs, in particular more than 100, more than 1 000, more than 2 000, more than 5 000 or more than 10 000 different UMIs.

In some embodiments, the unique molecular identifier comprises from 3 to 30 or more nucleotides. In some cases, the length of the unique molecular identifier may be 3, 4, 5, 6, 7, 8, 9, 10, 11, 12, 13, 14, 15, 16, 17, 18, 19, 20, 21, 22, 23, 24, 25, 26, 27, 28, 29, 30 nucleotides. In some cases, the length of the unique molecular identifier may be at least 3, 4, 5, 6, 7, 8, 9, 10, 11, 12, 13, 14, 15, 16, 17, 18, 19, 20, 21, 22, 23, 24, 25, 26, 27, 28, 29, 30 nucleotides or longer. In some cases, the length of the unique molecular identifier may be at most 3, 4, 5, 6, 7, 8, 9, 10, 11, 12, 13, 14, 15, 16, 17, 18, 19, 20, 21, 22, 23, 24, 25, 26, 27, 28, 29, 30 nucleotides or shorter.

In preferred embodiments, the length of the unique molecular identifier ranges from 5 to 25 nucleotides, preferably from 7 to 15 nucleotides, more preferably from 8 to 13 nucleotides.

In some embodiments, the oligonucleotide may thus comprise (i) a nucleic acid barcode and (ii) at least one, two or the three of a hybridization or probing sequence, a PCR handle sequence and a unique molecular identifier.

In some embodiments, the oligonucleotide may comprise, preferably in the recited order: (i) a PCR handle sequence, (ii) a nucleic acid barcode, (iii) a unique molecular identifier, and (iv) a hybridization or probing sequence.

According to the invention, the method of complexing biological units with particles is carried out in the presence of at least one means for binding a biological unit.

Such means for binding a biological unit include, but are not limited to, a nucleic acid (e.g., DNA, RNA, or artificial nucleic acids, such as nucleic acids comprising inosine, xanthosine, wybutosine, and/or analogs thereof), a lipid (e.g., a fatty acid, a glycerolipid, a glycerophospholipid, a sphingolipid, a saccharolipid, a polyketide, a sterol lipid and/or a prenol lipid), a protein or a fragment thereof, a peptide, an antibody (e.g., IgA, IgD, IgE, IgG, and IgM) or an antigen-binding fragment thereof (e.g., Fab fragments, F(ab')$_2$ fragments, scFv fragments, diabodies, triabodies, scFv-Fc fragments and/or minibodies), a carbohydrate (e.g., monosaccharides, disaccharides and/or polysaccharides), a vitamin or a derivative thereof, a coenzyme or a derivative thereof, a receptor ligand or derivative thereof and a hydrophobic group (e.g., alkyl groups having from about 2 to about 8 carbon atoms, such as an ethyl, propyl, butyl, pentyl, heptyl, or octyl and isomeric forms thereof; or aryl groups such as phenyl, benzyl or naphthyl).

The at least one means for binding the biological unit may be a direct means, i.e., a means coupling the particles directly (without necessitating an intermediary means) with the biological units. According to this embodiment, the means for binding the biological units is a molecule typically attached or otherwise bound to a particle, and interacting with a biological unit or part thereof. For the sake of illustration, the at least one means for binding the biological unit may be an antibody or a fragment thereof, or a receptor ligand, attached or otherwise bound to a particle, where the antibody would target and bind to a specific molecule or the ligand would target and bind to a specific receptor of the biological unit.

Alternatively, the at least one means for binding the biological unit may be an indirect means, *i.e.,* a means coupling the particles with the biological units through an intermediary means. According to this embodiment, the means for binding the biological units is typically a group of molecules capable of interacting together to form a bridge between a particle and a biological unit. Typically, at least one molecule of the group of molecules is attached or otherwise bound to a particle.

Such indirect means for binding the biological unit may be in particular a group of molecules comprising (i) a first nucleic acid molecule *(e.g.,* attached or otherwise bound to a particle), capable of hybridizing to (ii) a complementary nucleic acid fused to a biological unit-binding moiety, in particular a membrane-binding moiety when the biological unit comprises a lipid bilayer. In other words, the indirect means for binding the biological unit may comprise two hybridizable nucleic acid molecules, a first one being attached or otherwise bound to a particle and a second one being fused to biological unit-binding moiety.

Examples of biological unit-binding moiety or membrane-binding moieties include, but are not limited to, lipids, antibodies or antigen-binding fragments thereof, and receptors ligand or derivatives thereof.

The biological unit-binding moiety or membrane-binding moiety may be a lipid selected from the group consisting of fatty acids, glycerolipids, glycerophospholipids, sphingolipids, saccharolipids, polyketides, sterol lipids and prenol lipids. In some embodiments, the biological unit-binding moiety or membrane-binding moiety is a sterol lipid. Examples of sterol lipids include zoosterol lipids *(i.e.,* found in the membranes of animal cells) such as cholesterol; phytosterol lipids *(i.e.,* found in the membranes of plant cells) such as campesterol, sitosterol, and stigmasterol; and fungisterol lipids *(i.e.,* found in the membranes of fungi) such as ergosterol.

In some embodiments, the biological unit-binding moiety or membrane-binding moiety is cholesterol.

The biological unit-binding moiety or membrane-binding moiety may alternatively be an antibody or antigen-binding fragment thereof. In particular, the antibody or antigen-binding fragment thereof may be specific for a protein expressed at the surface of the biological unit.

In some embodiments, the method may therefore comprise an initial step of contacting the population of dissociated biological units with at least one biological unit-binding moiety or membrane-binding moiety fused to at least a first nucleic acid molecule. In this step, the biological unit is coated with the at least a first nucleic acid molecule.

The method may then comprise a second step of contacting the population of "coated" biological units with the population of particles, wherein each particle in the population of particles comprises at least a second nucleic acid molecule. In this step, the particles are coupled with the coated biological units through hybridization of the at least first nucleic acid molecule with the at least second nucleic acid molecule.

The at least first nucleic acid molecule and the at least second nucleic acid molecule should be capable of hybridization under standard conditions, in particular at any temperature ranging from about 4°C to about 42°C, from about 20°C to about 42°C, from about 20°C to about 37°C, from about 25°C to about 42°C, from about 25°C to about 37°C, or at room temperature.

The at least first nucleic acid molecule may be partially or fully complementary to the at least second nucleic acid molecule. The skilled artisan is readily capable of determining two partially complementary nucleic acid sequences which are capable of hybridization with each other under standard conditions.

In some embodiments, the at least first nucleic acid molecule comprises or consists of a poly(dA) sequence, and the at least second nucleic acid molecule comprises or consists of a poly(dT) or poly(U) sequence. In some embodiments, the at least first nucleic acid molecule comprises or consists of a poly(dT) or poly(U) sequence, and the at least second nucleic acid molecule comprises or consists of a poly(dA) sequence.

The at least first nucleic acid molecule and/or the at least second nucleic acid molecule may comprise from 5 to 300 or more nucleotides. In some cases, the length of the at least first nucleic acid molecule and/or of the at least second nucleic acid molecule may be 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides. In some cases, the length of the at least first nucleic acid molecule and/or of the at least second nucleic acid molecule may be at least 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides or longer. In some cases, the length of the at least first nucleic acid molecule and/or of the at least second nucleic acid molecule may be at most 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257, 258, 259, 260, 261, 262, 263, 264, 265, 266, 267, 268, 269, 270, 271, 272, 273, 274, 275, 276, 277, 278, 279, 280, 281, 282, 283, 284, 285, 286, 287, 288, 289, 290, 291, 292, 293, 294, 295, 296, 297, 298, 299, 300 nucleotides or shorter.

In some embodiments, the at least first nucleic acid molecule and the at least second nucleic acid molecule have the same length. In some embodiments, the at least first nucleic acid molecule and the at least second nucleic acid molecule do not have the same length.

In preferred embodiments, the length of the at least first nucleic acid molecule, that is fused with at least one biological unit-binding moiety or membrane binding moiety, ranges from 5 to 60 nucleotides, preferably from 15 to 50 nucleotides, more preferably from 25 to 50 nucleotides, even more preferably from 35 to 45 nucleotides. In these particular preferred embodiments, the biological unit-binding moiety or membrane-binding moiety may be a lipid as described above.

In preferred embodiments, the length of the at least first nucleic acid molecule, that is fused with at least one biological unit-binding moiety or membrane binding moiety, ranges from 50 to 300 nucleotides, preferably from 75 to 250 nucleotides, more preferably from 100 to 200 nucleotides. In these particular preferred embodiments, the biological unit-binding moiety or membrane-binding moiety may be an antibody or antigen-binding fragment thereof as described above.

In preferred embodiments, the length of the at least second nucleic acid molecule, that is comprised within each particle in the population of particles, ranges from 10 to 50 nucleotides, preferably from 15 to 45 nucleotides, more preferably from 20 to 40 nucleotides, even more preferably from 25 to 35 nucleotides.

The thickening agent increases the viscosity of the mix of biological units and particles during their coupling to form the suspension of biological unit-particle complexes.

In some embodiments, the thickening agent is selected from the group consisting of water-soluble polymers (such as, *e.g.,* agar, agarose, agaropectin, algin, alginate, bentonite, carbomer, carrageenan, cellulose, chitin, dextran, furacellaran, gelatin, gellan, hydroxyethylcellulose, mylopectin, pectin, polyacrylamide, polyacrylic acid, polyethylene glycol, polymethacrylic acid, polyvinyl alcohol, starch, xanthan, derivatives thereof and combinations thereof) and water-soluble non-polymer molecules (such as, *e.g.,* monosaccharides, disaccharides, glycerol, triacetin, ethylene glycol, derivatives thereof and combinations thereof).

In some embodiments, the thickening agent is agarose. In preferred embodiments, the thickening agent is agarose at a final concentration above (but not including) 0.2 % w/v and below (but not including) 2.5 % w/v, preferably at a final concentration ranging from 0.75 % to 1.5 % w/v. In preferred embodiments, the thickening agent is agarose at a final concentration of about 0.75 %, 0.8 %, 0.85 %, 0.9 %, 0.95 %, 1 %, 1.05 %, 1.1 %, 1.15 %, 1.2 %, 1.25 %, 1.3 %, 1.35 %, 1.4 %, 1.45 %, or 1.5 % w/v.

In some embodiments, the thickening agent is polyethylene glycol (PEG) or a derivative thereof, such as, *e.g.,* PEG 200, PEG 250 DME (dimethyl ether), PEG 350, PEG 400, PEG 500 DME, PEG 600, PEG 1 000, PEG 1 500, PEG 2 000 MME (monomethyl ether), PEG 4 000, PEG 5 000 MME, PEG 6 000, PEG 7 000, PEG 8 000, PEG 9 000, PEG 10 000, PEG 12 000, PEG 20 000, PEG 35 000, and the like.

In some embodiments, the thickening agent is PEG 8 000. In preferred embodiments, the thickening agent is PEG 8 000 at a final concentration above (but not including) 1 % w/v and below (but not including) 40 % w/v, preferably at a final concentration ranging from 5 % to 20 % w/v. In preferred embodiments, the thickening agent is PEG 8 000 at a final concentration of about 5 %, 5.5 %, 6 %, 6.5 %, 7.5 %, 8 %, 8.5 %, 9 %, 9.5 %, 10 %, 10.5 %, 11 %, 11.5 %, 12 %, 12.5 %, 13 %, 13.5 %, 14 %, 14.5 %, 15 %, 15.5 %, 16 %, 16.5 %, 17 %, 17.5 %, 18 %, 18.5 %, 19 %, 19.5 %, or 20 % w/v.

According to the invention, the thickening agent should be at a concentration sufficient to achieve a final viscosity *(i.e.,* the viscosity of the suspension of biological unit-particle complexes) ranging from about 3 mPa·s to about 15 mPa·s.

While it is known that viscosity changes with temperature and thickening agent concentration, the final viscosity should range from about 3 mPa·s to about 15 mPa·s at any given working temperature. This implies that the variable parameter is the concentration of the thickening agent used at the given working temperature to achieve a final viscosity ranging from about 3 mPa·s to about 15 mPa·s.

In some embodiments, the final viscosity of the suspension of biological unit-particle complexes ranges from about 3 mPa·s to about 15 mPa·s at any temperature ranging from about 4°C to about 42°C, from about 20°C to about 42°C, from about 20°C to about 37°C, from about 25°C to about 42°C, from about 25°C to about 37°C, or at room temperature. As used herein, room temperature ranges preferably from about 18°C to about 25°C, preferably from about 19°C to 23°C, more preferably from about 20°C to about 22°C.

In some embodiments, the final viscosity of the suspension of biological unit-particle complexes is about 3 $\pm$ 0.5 mPa·s, 4 $\pm$ 0.5 mPa·s, 5 $\pm$ 0.5 mPa·s, 6 $\pm$ 0.5 mPa·s, 7 $\pm$ 0.5 mPa·s, 8 $\pm$ 0.5 mPa·s, 9 $\pm$ 0.5 mPa·s, 10 $\pm$ 0.5 mPa·s, 11 $\pm$ 0.5 mPa·s, 12 $\pm$ 0.5 mPa·s, 13 $\pm$ 0.5 mPa·s, 14 $\pm$ 0.5 mPa·s, or 15 $\pm$ 0.5 mPa·s.

In some embodiments, the final viscosity is not 5.0 mPa·s or 10.0 mPa·s, in particular, the final viscosity is not 5.0 mPa·s or 10.0 mPa·s at 25°C.

The method of complexing biological units with particles described herein allows to obtain a suspension of biological unit-particle complexes comprising less than 50 % of uncomplexed (or uncoupled) biological units *(i.e.,* less than 50 % of the biological units initially present in the population of dissociated biological units are not complexed (or coupled) with at least one particle).

The method of complexing biological units with particles described herein further allows to obtain a suspension of biological unit-particle complexes comprising less than 10 % of the biological units initially present in the population of dissociated biological units which are complexed (or coupled) with a particle concurrently with one or several other biological units *(i.e.,* complexes comprising 2 or more biological units bound to 1 or more particles).

The method of complexing biological units with particles described herein may be implemented for a variety of applications, in particular in the field of single-cell analysis.

In particular, the method of complexing biological units with particles described herein may be implemented with any of the methods described in WO 2018/203141, the content of which is incorporated herein by reference.

One application of the method of complexing biological units with particles described herein is a method of trapping biological unit-particle complexes in a hydrogel, said method comprising or consisting of the following steps:

a) complexing biological units with particles according to the method described herein, to form a suspension of biological unit-particle complexes, and

b) forming a hydrogel matrix trapping the biological unit-particle complexes, preferably in a discrete fashion.

By **"discrete",** it is meant spatially dispersed and distant from each other. In some embodiments, two biological unit-particle complexes are considered as discrete when they are not in physical contact with each other. In particular, two biological unit-particle complexes can be considered as discrete when the smallest distance between the two is larger than 0.5 times the mean radius of the complex, preferably 1 time, 2 times, 5 times, 10 times, 20 times, 50 times or more the mean radius of the complex.

In some embodiments, the method of trapping biological unit-particle complexes in a hydrogel comprises or consists of the following steps:

a) complexing biological units with particles according to the method described herein, to form a suspension of biological unit-particle complexes,

b) diluting the suspension of biological unit-particle complexes, and

c) forming a hydrogel matrix trapping the biological unit-particle complexes, preferably in a discrete fashion.

The step of diluting the suspension of biological unit-particle complexes may be desirable to achieve a desired level of discreteness of the biological unit-particle complexes trapped in the hydrogel. The skilled artisan can readily determine if and to what extent the suspension should be diluted.

In some embodiments, the suspension of biological unit-particles complexes is diluted to a final concentration ranging from 1 to 50 biological units per μL, preferably from 1 to 25 biological units per μL, more preferably from 1 to 10 biological units per μL. In some embodiments, the suspension of biological unit-particles complexes is diluted to a final concentration of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49 or 50 biological unit per μL.

In some embodiments, the suspension of biological unit-particle complexes is diluted with at least one density-matching agent. By "density-matching agent", it is meant an agent which is capable of maintaining a neutral buoyancy in the suspension of biological unit-particle complexes. Neutral buoyancy occurs when an object's average density (*e.g.,* the biological unit-particle complexes) is equal to the density of the fluid in which it is immersed, resulting in the buoyant force balancing the force of gravity that would otherwise cause the object to float or sediment.

The use of density-matching agents is of particular interest to enable a stable distribution of biological unit-particle complexes in their suspension, and to avoid - or at least lessen - their sedimentation or flotation and their progressive gathering at the top or the bottom of the suspension, which could otherwise reduce the discreetness of the biological units and favor the formation of high order biological unit-particle complexes (*e.g.,* complexes comprising several biological units complexed with one particle and/or several particles complexed with one biological unit) and/or of aggregates of biological unit-particle complexes.

Examples of density-matching agents include, but are not limited to, iodixanol (*e.g.,* OptiPrep™), iohexol (*e.g.,* Accudenz™, Histodenz™, Nycodenz™), sucrose, sucrose acetate isobutyrate, polysucrose (*e.g.,* alginate or Ficoll®), calcium phosphate, colloidal silica (*e.g.,* Percoll®), ester gum, damar gum, brominated vegetable oil, sodium metatungstate and combinations thereof.

In some embodiments, the density-matching agent is iodixanol. For instance, iodixanol is commercially available under the tradename OptiPrep™, which is a non-ionic aqueous solution containing 60 % w/v of iodixanol and having a volumetric mass density of 1.320 kg/m³.

In preferred embodiments, the density-matching agent is iodixanol at a final concentration ranging from 20 % to 60 % iodixanol, preferably from 30 % to 50 % iodixanol, more preferably from 35 % to 45 % iodixanol. In preferred embodiments, the density-matching agent is or comprises iodixanol at a final concentration of 40% iodixanol.

In some embodiments, the method of trapping biological unit-particle complexes in a hydrogel comprises or consists of the following steps:

a) complexing biological units with particles according to the method described herein, to form a suspension of biological unit-particle complexes,
b) diluting the suspension of biological unit-particle complexes with a gellable solution, and
c) allowing the gellable solution to solidify to form a hydrogel matrix trapping the biological unit-particle complexes, preferably in a discrete fashion.

Examples of gellable solutions have been described herein, and include, without limitation, polysaccharide gellable solutions (such as, *e.g.,* alginate, agarose, κ-carrageenan, ι-carrageenan, chitosan, dextran, dialdehyde starch, heparin, gellan, native gellan gum, rhamsan, deacetylated rhamsan, S-657, xanthan gum and welan solutions), protein-based gellable solutions (such as, *e.g.,* collagen, fibrin, albumin, gelatin, and laminin solutions), and combinations thereof.

In some embodiments, the gellable solution is a solution of agarose (including low-melting point agarose), dextran/polyethylene glycol (PEG)/polyvinyl alcohol (PVA) (commercially available under the tradename TrueGel3D™), alginate, or xanthan.

In preferred embodiments, the gellable solution is a solution of agarose, preferably at a final concentration ranging from 0.5 % to 5 %, more preferably from 0.5 % to 2.5 %. In some embodiments, agarose is hydroxyethyl agarose (commercially available under the tradename SeaPrep® agarose), preferably at a final concentration ranging from 0.5 % to 5 %, more preferably from 0.5 % to 2.5 %, even more preferably at a final concentration of 1.5 %.

The at least one thickening agent used in the method of complexing biological units with particles as described herein may be a gellable solution. For illustration purposes, agarose can be a suitable thickening agent, while being gellable and capable of forming a hydrogel at the same time. Hence, the skilled artisan will readily understand that the method of trapping biological unit-particle complexes in a hydrogel will include a step of diluting the suspension of biological unit-particle complexes with a gellable solution, in particular when the at least one thickening agent was not itself a gellable solution.

In some embodiments, the method of trapping biological unit-particle complexes in a hydrogel comprises or consists of the following steps:

a) complexing biological units with particles according to the method described herein, to form a suspension of biological unit-particle complexes,
b) diluting the suspension of biological unit-particle complexes with a gellable solution and at least one density-matching agent, and
c) allowing the gellable solution to solidify to form a hydrogel matrix trapping the biological unit-particle complexes, preferably in a discrete fashion.

The method of trapping biological unit-particle complexes in a hydrogel described herein can be a starting point for analysis processes at the single biological unit level.

For instance, one of such analysis process can be a method of uniquely identifying a biological unit's analytes, said method comprising or consisting of the following steps:

> a) complexing biological units with particles according to the method described herein, to form a suspension of biological unit-particle complexes,
> wherein each particle comprises a unique identifier or clonal copies thereof,
> b) forming a hydrogel matrix trapping the biological unit-particle complexes, preferably in a discrete fashion, and
> c) labelling the biological unit's analytes of each biological unit-particle complex with the unique identifier.

In some embodiments, the biological unit's analytes are nucleic acids, such as, without limitation, DNA (including genomic DNA and organellar DNA, *i.e.*, mitochondrial DNA or chloroplast DNA) or RNA (including messenger RNA, transfer RNA, ribosomal RNA, transfer-messenger RNA, microRNA, small interfering RNA, Piwi-interacting RNA, antisense RNA, small nuclear RNA, small nucleolar RNA, small Cajal body RNA and enhancer RNA).

In some embodiments, the biological unit's analytes are messenger RNAs.

By **"labelling the biological unit's analytes"**, it is meant attaching or linking, either covalently or non-covalently, the unique identifier to the analytes. In some embodiments, the unique identifier is a nucleic acid barcode, as already described above; **"labelling the biological unit's analytes"** may thus comprise, *e.g.*, annealing, hybridizing, ligating, etc. the nucleic acid barcode to the analytes.

In some embodiments, the step of labelling the biological unit's analytes comprises a substep of lysing the biological units or otherwise releasing the analytes from the biological units.

In some embodiments, lysing the biological units or otherwise releasing the analytes from the biological units includes disrupting, permeabilizing or solubilizing the lipid membrane (*e.g.*, the lipid bilayer) of the biological unit.

Means and methods for lysing the biological units or otherwise releasing analytes from biological units are well-known in the art. Preferably, the method is chosen among non-mechanical ones. These include, but are not limited to, chemical lysis (*e.g.*, using one or a combination of a detergent, a chaotropic agent, an alkaline agent, a solvent, etc.), physical lysis *(e.g.,* by one or a combination of heating, freezing/thawing cycles, by osmotic shock, by cavitation, etc.), and enzymatic lysis (*e.g.*, using enzymes).

In some embodiments, the step of labelling the biological unit's analytes, including, when applicable, the substep of lysing the biological units or otherwise releasing the analytes from the biological units is carried out in the hydrogel matrix.

In some embodiments, the method of uniquely identifying a biological unit's analytes comprises or consists of the following steps:

> a) complexing biological units with particles according to the method described herein, to form a suspension of biological unit-particle complexes,
> wherein each particle comprises a unique identifier or clonal copies thereof,
> b) diluting the suspension of biological unit-particle complexes,
> c) forming a hydrogel matrix trapping the biological unit-particle complexes, preferably in a discrete fashion, and
> d) labelling the biological unit's analytes of each biological unit-particle complex with the unique identifier.

In some embodiments, the method of uniquely identifying a biological unit's analytes comprises or consists of the following steps:

> a) complexing biological units with particles according to the method described herein, to form a suspension of biological unit-particle complexes,
> wherein each particle comprises a unique identifier or clonal copies thereof,
> b) diluting the suspension of biological unit-particle complexes with a gellable solution, and
> c) allowing the gellable solution to solidify to form a hydrogel matrix trapping the discrete biological unit-particle complexes, and
> d) labelling the biological unit's analytes of each biological unit-particle complex with the unique identifier.

In some embodiments, the method of uniquely identifying a biological unit's analytes comprises or consists of the following steps:

> a) complexing biological units with particles according to the method described herein, to form a suspension of biological unit-particle complexes,
> wherein each particle comprises a unique identifier or clonal copies thereof,

b) diluting the suspension of biological unit-particle complexes with a gellable solution and at least one density-matching agent, and

c) allowing the gellable solution to solidify to form a hydrogel matrix trapping the discrete biological unit-particle complexes, and

d) labelling the biological unit's analytes of each biological unit-particle complex with the unique identifier.

Analyses can then be performed on the biological unit's analytes once they are labelled with their unique identifier, which allows to trace back a given analyte to its original biological unit.

A first analysis that can be performed on the biological unit's analytes is gene expression analysis in single biological units.

Single-cell gene expression analysis, or transcriptome profiling, relies on the amplification of a single-cell's mRNA content and its sequencing. The generation of a single-cell transcriptome generally requires a first step of reverse transcription to convert the mRNAs with poly(dA) tails into cDNAs, which can be further amplified and sequenced.

The present invention thus relates to a method of analyzing gene expression in single biological units.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) forming a hydrogel matrix trapping the biological unit-particle complexes, preferably in a discrete fashion,
c) labelling the biological unit's analytes of each biological unit-particle complex with the unique identifier,
d) sequencing the biological unit's analytes labelled with their unique identifier.

As explained above, the biological unit's analytes may be nucleic acids, in particular DNA or RNA, and preferably the biological unit's analytes are messenger RNAs.

As explained above, the unique identifier may be a nucleic acid barcode.

As explained above, the step of labelling the biological unit's analytes may comprise a substep of lysing the biological units or otherwise releasing the analytes from the biological units. As explained above, this step of labelling the biological unit's analytes, including, when applicable, the substep of lysing the biological units or otherwise releasing the analytes from the biological units) may be carried out in the hydrogel matrix.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes,
c) forming a hydrogel matrix trapping the biological unit-particle complexes, preferably in a discrete fashion,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution,
c) allowing the gellable solution to solidify to form a hydrogel matrix trapping the discrete biological unit-particle complexes,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution and at least one density-matching agent,

c) allowing the gellable solution to solidify to form a hydrogel matrix trapping the discrete biological unit-particle complexes,

d) labelling the biological unit's analytes of each biological unit-particle complex with the unique identifier,

e) sequencing the biological unit's analytes labelled with their unique identifier.

In some embodiments, the final step of sequencing the biological unit's analytes labelled with their unique identifier may be preceded by one or several additional steps well-known in the art, including, without limitation:

- a step of reverse transcribing the biological unit's analytes (in particular the biological unit's RNA) labelled with their unique identifier, including a first- and second-strand synthesis step, thereby obtaining a DNA molecule comprising (i) the unique identifier and (ii) the partial or complete complementary sequence of the biological unit's analytes; and/or
- a step of amplifying the biological unit's analytes labelled with their unique identifier, thereby obtaining a DNA molecule comprising (i) the unique identifier and (ii) the sequence of the biological unit's analytes; and/or
- a step of purifying the biological unit's analytes labelled with their unique identifier, and/or
- a step of preparing a sequencing library from the biological unit's analytes labelled with their unique identifier.

Other additional steps include steps of washing, buffer exchange, exonuclease treatment, etc.

These additional steps and others are well-known to the skilled artisan. Such steps are described in Macosko et al., 2015. Cell. 161:1202-1214; Fan et al., 2015. Science. 347(6222):1258367; Klein et al., 2015. Cell. 161(5):1187-201; Gierahn et al., 2017. Nat Methods. 14(4):395-398; and US patent applications US 2016-0289669, US 2016-0265069, US 2016-0060621 and US 2015-0376609, the content of all of which is incorporated herein by reference.

In particular, when the step of labelling the biological unit's analytes of each biological unit-particle complex with the unique identifier comprises annealing or hybridizing a unique identifier *(e.g.,* a nucleic acid barcode) to the analytes, it may be desirable to perform a subsequent step of reverse transcription and/or amplification of the analytes in order to stably incorporate the unique identifier in the analyte.

In some embodiments, the final step of sequencing the biological unit's analytes labelled with their unique identifier is performed in solution, *i.e.,* not in the hydrogel matrix. In other words, this step can be preceded by a step of depolymerizing the hydrogel matrix. In any case, the step of depolymerizing the hydrogel matrix should be carried out after the step of labelling the biological unit's analytes of each biological unit-particle complex with the unique identifier, in order to ensure that the analytes can be traced back to their original biological unit, even when mixed in solution altogether. This step of depolymerizing the hydrogel matrix can also be carried out at any later stage, *e.g.,* after a step of reverse transcription, of amplification, of sequencing library preparation, etc.

Means and methods for depolymerizing a hydrogel matrix are well-known in the art; in particular, the skilled artisan is aware that the means and methods should be adapted to the hydrogel matrix. Some non-limitative examples of such means and methods include the use of chaotropic agents (such as guanidine, urea, lithium perchlorate, etc. and their salts), the change of ionic concentration (*e.g.,* when the hydrogel matrix is composed of alginate), the use of reducing agents (such as phosphines or DTT; *e.g.,* when the hydrogel matrix is albumin), the change of temperature (such as a temperature increase; *e.g.,* when the hydrogel matrix is thermosensitive or thermoreversible), and the use of enzymes (*e.g.,* agarose, when the hydrogel matrix is agarose).

An exemplary protocol for a method of analyzing gene expression in single biological units implementing the methods described herein is detailed in Example 5.

Another analysis that can be performed on the biological unit's analytes is genotyping in single biological units.

Single-cell genotyping relies on the whole genome amplification (WGA) of a single-cell's DNA to generate enough DNA for sequencing. Several methods for WGA are available and well-known to the skilled artisan. Some methods however lead to amplification bias, and subsequent inadequate genome coverage. PCR-based exponential WGA with degenerate primers introduces sequence-dependent bias. Multiple displacement amplification (MDA), using the strand-displacing Φ29 DNA polymerase, represents an improvement, but may also introduce bias due to nonlinear amplification. Multiple annealing and loop-based amplification cycles (MALBAC) is another method which introduces quasilinear preamplification to reduce the bias associated with nonlinear amplification. It relies on the BstI DNA polymerase for the quasilinear preamplification phase, along with high-fidelity PCR enzymes for subsequent exponential amplification (Zong et al., 2012. Science. 338(6114):1622-6; Lu et al., 2012. Science. 338(6114):1627-30).

The present invention thus also relates to a method of analyzing the genotype in single biological units.

In some embodiments, the method comprises or consists of the following steps:

a) complexing biological units with particles according to the method described herein, to form a suspension of biological unit-particle complexes,

wherein each particle comprises a unique identifier or clonal copies thereof,

b) forming a hydrogel matrix trapping the biological unit-particle complexes, preferably in a discrete fashion,
c) labelling the biological unit's analytes of each biological unit-particle complex with the unique identifier,
d) sequencing the biological unit's analytes labelled with their unique identifier.

As explained above, the biological unit's analytes may be nucleic acids, in particular DNA or RNA, and preferably the biological unit's analytes are genomic DNA.

As explained above, the unique identifier may be a nucleic acid barcode.

As explained above, the step of labelling the biological unit's analytes comprises a substep of lysing the biological units or otherwise releasing the analytes from the biological units. As explained above, this step of labelling the biological unit's analytes, including, when applicable, the substep of lysing the biological units or otherwise releasing the analytes from the biological units) may be carried out in the hydrogel matrix.

As explained above, the final step of sequencing the biological unit's analytes labelled with their unique identifier is performed in solution, *i.e.,* not in the hydrogel matrix; hence, this step can be preceded by a step of depolymerizing the hydrogel matrix.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes,
c) forming a hydrogel matrix trapping the biological unit-particle complexes, preferably in a discrete fashion,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution,
c) allowing the gellable solution to solidify to form a hydrogel matrix trapping the discrete biological unit-particle complexes,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution and at least one density-matching agent,
c) allowing the gellable solution to solidify to form a hydrogel matrix trapping the discrete biological unit-particle complexes,
d) labelling the biological unit's analytes of each biological unit-particle complex with the unique identifier,
e) sequencing the biological unit's analytes labelled with their unique identifier.

As explained above, the final step of sequencing the biological unit's analytes labelled with their unique identifier may be preceded by one or several additional steps. These additional steps are well-known to the skilled artisan. Such steps are described in Hutchison et al., 2005. Proc Natl Acad Sci USA. 102(48):17332-6; Leung et al., 2016. Proc Natl Acad Sci USA. 113(30):8484-9; Wang et al., 2012. Cell. 150(2):402-12; Marcy et al., 2007. PLoS Genet. 3(9):1702-8; Gole et al., 2013. Nat Biotechnol. 31(12):1126-32; Zhang et al., 2006. Nat Biotechnol. 24(6):680-6; and in International applications WO 2016/061517 and WO 2005/003304, the content of all of which is incorporated herein by reference.

In some embodiments, the final step of sequencing the biological unit's analytes labelled with their unique identifier may be preceded by one or several additional steps well-known in the art, including, without limitation:

- a step of synthesis of a complementary sequence of the biological unit's analytes (in particular the biological unit's DNA) labelled with their unique identifier by extension, thereby obtaining a DNA molecule comprising (i) the unique identifier and (ii) the partial or complete complementary sequence of the biological unit's analytes; and/or
- a step of amplifying the biological unit's analytes labelled with their unique identifier, thereby obtaining a DNA molecule comprising (i) the unique identifier and (ii) the sequence of the biological unit's analytes; and/or
- a step of purifying the biological unit's analytes labelled with their unique identifier, and/or
- a step of preparing a sequencing library from the biological unit's analytes labelled with their unique identifier.

When analyzing the genotype in single biological units, it is also possible to analyze their haplotype, *e.g.,* for phasing.

Phasing relies on the whole genome amplification (WGA) of a high molecular weight, *i.e.,* greater than 25 or 50 kilobases, DNA to generate enough DNA for sequencing. Several methods for WGA are available and well-known to the skilled artisan. Some methods however lead to amplification bias, and subsequent inadequate genome coverage. PCR-based exponential WGA with degenerate primers introduces sequence-dependent bias. Multiple displacement amplification (MDA), using the strand-displacing Φ29 DNA polymerase, represents an improvement, but may also introduce bias due to nonlinear amplification. Multiple annealing and loop-based amplification cycles (MALBAC) is another method which introduces quasilinear preamplification to reduce the bias associate with nonlinear amplification. It relies on the BstI DNA polymerase for the quasilinear preamplification phase, along with high-fidelity PCR enzymes for subsequent exponential amplification (Zong et al., 2012. Science. 338(6114):1622-6; Lu et al., 2012. Science. 338(6114):1627-30). Alternatively, the Tn5 transposase and subsequent amplification can be used for library prep in a method termed "Contiguity-Preserving Transposition" (CPT-seq) (Amini et al., 2014. Nat Genet. 46(12):1343-9). In this method, the first step after the genomic DNA has been optionally purified is to tagment the DNA through Tn5 transposition. This fragments the DNA and adds universal adaptors directly to the template. After gap filling, PCR then occurs using primers complementary to the inserted Tn5 adaptors followed by sequencing.

The method, when for phasing, may comprise one or several additional steps which are well-known to the skilled artisan. Such steps are described in International applications WO 2015/126766, WO 2016/130704, WO 2016/61517, WO 2015/95226, WO 2016/003814, WO 2005/003304, WO 2015/200869, WO 2014/124338, WO 2014/093676; US patent application US 2015-066385; Kuleshov et al., 2014. Nat Biotechnol. 32(3):261-6; Amini et al., 2014. Nat Genet. 46(12):1343-9; Kaper et al., 2013. Proc Natl Acad Sci USA. 110(14):5552-7; Peters et al., 2012. Nature. 487(7406):190-5 and Zheng et al., 2016. Nat Biotechnol. 34(3):303-11, the content of all of which is incorporated herein by reference.

Another analysis that can be performed on the biological unit's analytes is epigenome analysis in single biological units.

Single-cell nucleosome positioning based on Tn5 transposition has been developed, termed "Assay for Transposase-Accessible Chromatin with high throughput sequencing" (ATAC-seq) (Buenrostro et al., 2015. Nature. 523(7561):486-90). In this method, the first step enables molecular access to nucleosome-free DNA by using low-percentage non-ionic detergents on intact cells or isolated nuclei. The accessible DNA is then tagmented through Tn5 transposition. This fragments the DNA and adds universal adaptors directly to the template. PCR then occurs using primers complementary to those adaptors followed by sequencing.

The present invention thus also relates to a method of analyzing the epigenome in single biological units.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) forming a hydrogel matrix trapping the biological unit-particle complexes, preferably in a discrete fashion,
c) labelling the biological unit's analytes of each biological unit-particle complex with the unique identifier,
d) sequencing the biological unit's analytes labelled with their unique identifier.

As explained above, the biological unit's analytes may be nucleic acids, in particular DNA or RNA, and preferably the biological unit's analytes are non-nucleosomal DNA (*i.e.*, free DNA, which is not associated with histones).

As explained above, the unique identifier may be a nucleic acid barcode.

As explained above, the step of labelling the biological unit's analytes comprises a substep of lysing the biological units or otherwise releasing the analytes from the biological units. As explained above, this step of labelling the biological unit's analytes, including, when applicable, the substep of lysing the biological units or otherwise releasing the analytes from the biological units) may be carried out in the hydrogel matrix.

As explained above, the final step of sequencing the biological unit's analytes labelled with their unique identifier is performed in solution, *i.e.,* not in the hydrogel matrix; hence, this step can be preceded by a step of depolymerizing the hydrogel matrix.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes,
c) forming a hydrogel matrix trapping the biological unit-particle complexes, preferably in a discrete fashion,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution,
c) allowing the gellable solution to solidify to form a hydrogel matrix trapping the discrete biological unit-particle complexes,
d) labelling the biological unit's analytes of each biological unit-particle complex with the unique identifier,
e) sequencing the biological unit's analytes labelled with their unique identifier.

In some embodiments, the method comprises or consists of the following steps:

a) complexing biological units with particles according to the method described herein, to form a suspension of biological unit-particle complexes,
wherein each particle comprises a unique identifier or clonal copies thereof,
b) diluting the suspension of biological unit-particle complexes with a gellable solution and at least one density-matching agent,
c) allowing the gellable solution to solidify to form a hydrogel matrix trapping the discrete biological unit-particle complexes,
d) labelling the biological unit's analytes of each biological unit-particle complex with the unique identifier,
e) sequencing the biological unit's analytes labelled with their unique identifier.

As explained above, the final step of sequencing the biological unit's analytes labelled with their unique identifier may be preceded by one or several additional steps. These additional steps are well-known to the skilled artisan. Such steps are described in International application WO 2014/189957; Buenrostro et al., 2015. Nature. 523(7561):486-90; Buenrostro et al., 2013. Nat Methods. 10(12):1213-8; and Christiansen et al., 2017. Methods Mol Biol. 1551:207-221, the content of all of which is incorporated herein by reference
The present invention also relates to a kit, in particular, a kit suitable for implementing the method of complexing biological units with particles described herein.
In some embodiments, the kit comprises:

- a first container or vial containing at least a first nucleic acid molecule fused to a biological unit-binding moiety, in particular a membrane-binding moiety, as defined above; and
- a second container or vial containing a population of particles, as defined above, said particles comprising at least a second nucleic acid molecule;

wherein the at least first nucleic acid molecule and the at least second nucleic acid molecule are capable of hybridization under standard conditions, in particular wherein the at least first nucleic acid molecule and the at least second nucleic acid molecule are partially or fully complementary to each other.
In some embodiments, the kit comprises:

- a first container or vial containing at least a first nucleic acid molecule fused to a lipid moiety; and
- a second container or vial containing a population of particles, as defined above, said particles comprising at least a second nucleic acid molecule,

wherein the at least first nucleic acid molecule and the at least second nucleic acid molecule are capable of hybridization under standard conditions, in particular wherein the at least first nucleic acid molecule and the at least second nucleic acid molecule are partially or fully complementary to each other.
In some embodiments, the kit comprises:

- a first container or vial containing at least a first nucleic acid molecule fused to an antibody or antigen-binding fragment thereof; and
- a second container or vial containing a population of particles, as defined above, said particles comprising at least a second nucleic acid molecule,

wherein the at least first nucleic acid molecule and the at least second nucleic acid molecule are capable of hybridization under standard conditions, in particular wherein the at least first nucleic acid molecule and the at least second nucleic acid molecule are partially or fully complementary to each other.

In some embodiments, the kit comprises:

- a first container or vial containing a poly(dA) molecule fused to a lipid moiety; and
- a second container or vial containing a population of particles bearing poly(dT) nucleic acids on their surface.

In some embodiments, the kit comprises:

- a first container or vial containing a poly(dA) molecule fused to an antibody or antigen-binding fragment thereof; and
- a second container or vial containing a population of particles bearing poly(dT) nucleic acids on their surface.

In some embodiments, the lipid moiety is capable of anchorage in the biological units' lipid membrane, as described herein.

In some embodiments, the kit comprises:

- a first container or vial containing at least a first nucleic acid molecule fused to a cholesterol moiety; and
- a second container or vial containing a population of particles, as defined above, said particles comprising at least a second nucleic acid molecule,

wherein the at least first nucleic acid molecule and the at least second nucleic acid molecule are capable of hybridization under standard conditions, in particular wherein the at least first nucleic acid molecule and the at least second nucleic acid molecule are partially or fully complementary to each other.

In some embodiments, the kit comprises:

- a first container or vial containing cholesteryl-poly(dA); and
- a second container or vial containing a population of particles bearing poly(dT) nucleic acids on their surface.

In some embodiments, the second container contains the population of particles in solution with at least one thickening agent, as defined above. Alternatively, the kit comprises a third container or vial containing the at least one thickening agent.

In some embodiments, the thickening agent is selected from the group consisting of water-soluble polymers (such as, *e.g.,* agar, agarose, agaropectin, algin, alginate, bentonite, carbomer, carrageenan, cellulose, chitin, dextran, furacellaran, gelatin, gellan, hydroxyethylcellulose, mylopectin, pectin, polyacrylamide, polyacrylic acid, polyethylene glycol, polymethacrylic acid, polyvinyl alcohol, starch, xanthan, derivatives thereof and combinations thereof) and water-soluble non-polymer molecules (such as, *e.g.,* monosaccharides, disaccharides, glycerol, triacetin, ethylene glycol, derivatives thereof and combinations thereof).

In some embodiments, the thickening agent is a water-soluble polymer. In some embodiments, the thickening agent is not a water-soluble non-polymer molecule. In some embodiments, the thickening agent is not glycerol.

In some embodiments, the thickening agent is polyethylene glycol (PEG) or a derivative thereof, such as, *e.g.,* PEG 200, PEG 250 DME (dimethyl ether), PEG 350, PEG 400, PEG 500 DME, PEG 600, PEG 1 000, PEG 1 500, PEG 2 000 MME (monomethyl ether), PEG 4 000, PEG 5 000 MME, PEG 6 000, PEG 7 000, PEG 8 000, PEG 9 000, PEG 10 000, PEG 12 000, PEG 20 000, PEG 35 000, and the like.

In preferred embodiments, the thickening agent is agarose or PEG 8 000.

In preferred embodiments, the thickening agent is present in the second container or vial, or alternatively in a third container or vial, at a viscosity sufficiently high to achieve a final viscosity ranging from about 3 mPa·s to about 15 mPa·s when diluted with water by a factor $1.1 \times$, $1.2 \times$, $1.3 \times$, $1.4 \times$, $1.5 \times$, $2 \times$, $2.5 \times$, $3 \times$, $4 \times$, $5 \times$ or more.

In preferred embodiments, the thickening agent is present in the second container or vial, or alternatively in a third container or vial, leading to a viscosity of at least $3 \pm 0.5$ mPa·s, preferably at least $4 \pm 0.5$ mPa·s, $5 \pm 0.5$ mPa·s, $6 \pm 0.5$ mPa·s, $7 \pm 0.5$ mPa·s, $8 \pm 0.5$ mPa·s, $9 \pm 0.5$ mPa·s, $10 \pm 0.5$ mPa·s, $11 \pm 0.5$ mPa·s, $12 \pm 0.5$ mPa·s, $13 \pm 0.5$ mPa·s, $14 \pm 0.5$ mPa·s, $15 \pm 0.5$ mPa·s or more.

In some embodiments, each particle in the population of particles comprises a unique identifier or clonal copies thereof, such as a nucleic acid barcode, as defined above.

In some embodiments, the kit may further comprise a container or a vial comprising a gellable solution, as defined above; and optionally at least one density-matching agent, as defined above. Alternatively, the kit may further comprise a container or a vial comprising a gellable solution, as defined above, and another container or vial comprising a density-matching agent, as defined above.

In some embodiments, the kit may further comprise additional containers or vials comprising reagents and solutions for biochemistry and molecular biology assays. Some examples of such reagents and solutions are described in the "Materials" section of Example 5.

In some embodiments, the additional containers or vials may comprise one or several of the following:

- a biological unit wash buffer; it is in particular desirable that the biological unit wash buffer be an isotonic buffer in order to avoid unwanted lysis of the biological units; it is also desirable that the biological unit wash buffer do not contain any nutrient in order to avoid any unwanted metabolic activity of the biological units;
- a lysis buffer; the lysis buffer may comprise one or several salts, pH buffers and chelating agents, as well as one or several of a detergent, a chaotropic agent, an alkaline agent, a solvent, or an enzyme; it is desirable that the detergent, chaotropic agent, alkaline agent, solvent or enzyme be small enough to diffuse through the 3-D network of a hydrogel matrix, e.g., that their molar mass be equal to or smaller than about 1 000 g/mol;
- a hydrogel depolymerization buffer; the hydrogel depolymerization buffer may comprise one or several of a chaotropic agent, a salt, a reducing agent, or an enzyme.
- a particle wash buffer;
- a reverse transcriptase (or RNA-directed DNA polymerase), as defined above;
- a reverse transcription buffer; the reverse transcription buffer may in particular comprise one or several salts and pH buffers, as well as a mix of the 4 deoxyribonucleotide triphosphate (dNTP); it may further comprise template switch oligos (TSO); it my further comprise a ribonuclease (RNAse) inhibitor;
- a DNA polymerase, as defined above;
- a PCR buffer; the PCR buffer may in particular comprise one or several salts and pH buffers, as well as a mix of the 4 deoxyribonucleotide triphosphate (dNTP); it may further comprise SMART™ PCR primers, *e.g.*, with SEQ ID NO: 1;
- an exonuclease;
- a SDS buffer;
- a Tween-20 buffer;
- library preparation primers;
- sequencing primers.

In some preferred embodiments, the kit further comprises at least a hydrogel depolymerization buffer. In some preferred embodiments, the kit further comprises at least a lysis buffer. In some preferred embodiments, the kit further comprises at least a reverse transcriptase and its reverse transcription buffer.

In some embodiments, the kit may further comprise a gelation device comprising a cap, a tube and a piston, as described in European patent application EP 22 305 071.7, the content of which is incorporated herein by reference.

The present invention also relates to a kit, in particular, a kit suitable for implementing the method of complexing biological units with particles described herein.

In some embodiments, the kit comprises:

- a first container or vial containing a population of particles and at least one means or a portion thereof for binding a biological unit, as defined above; and
- at least one thickening agent, either in a second container or vial, or in the first container together with the population of particles.

In some embodiments, the at least one means or a portion thereof for binding a biological unit is bound or otherwise attached to the particles of the population of particles.

In some embodiments where the first container or vial contains only a portion of the at least one means for binding a biological unit, the kit may further comprise a container or a vial comprising the remainder of the at least one means for binding a biological unit. For instance, the first container or vial may contain only at least one molecule of the group of molecules capable of interacting together to form a bridge between a particle and a biological unit; the remainder of the group of molecules being provided in a separate container or vial. Such means for binding a biological unit have been described herein and referred to as "indirect means".

According to this embodiment, a portion of the at least one means for binding a biological unit may be a nucleic acid molecule, and the remainder of the at least one means for binding a biological unit may be another nucleic acid molecule fused to a biological unit-binding moiety, wherein the two nucleic acid molecules are capable of hybridization under standard conditions, in particular wherein the two nucleic acid molecules are partially or fully complementary to each other, as was described herein.

In some embodiments, the thickening agent is selected from the group consisting of water-soluble polymers (such as, *e.g.,* agar, agarose, agaropectin, algin, alginate, bentonite, carbomer, carrageenan, cellulose, chitin, dextran, furacellaran, gelatin, gellan, hydroxyethylcellulose, mylopectin, pectin, polyacrylamide, polyacrylic acid, polyethylene glycol, polymethacrylic acid, polyvinyl alcohol, starch, xanthan, derivatives thereof and combinations thereof) and water-soluble non-polymer molecules (such as, *e.g.,* monosaccharides, disaccharides, glycerol, triacetin, ethylene glycol, derivatives thereof and combinations thereof).

In some embodiments, the thickening agent is a water-soluble polymer. In some embodiments, the thickening agent is not a water-soluble non-polymer molecule. In some embodiments, the thickening agent is not glycerol.

In some embodiments, the thickening agent is polyethylene glycol (PEG) or a derivative thereof, such as, *e.g.,* PEG 200, PEG 250 DME (dimethyl ether), PEG 350, PEG 400, PEG 500 DME, PEG 600, PEG 1 000, PEG 1 500, PEG 2 000 MME (monomethyl ether), PEG 4 000, PEG 5 000 MME, PEG 6 000, PEG 7 000, PEG 8 000, PEG 9 000, PEG 10 000, PEG 12 000, PEG 20 000, PEG 35 000, and the like.

In preferred embodiments, the thickening agent is agarose or PEG 8 000.

In preferred embodiments, the thickening agent is present in the first container or vial, or alternatively in a second container or vial, at a viscosity sufficiently high to achieve a final viscosity ranging from about 3 mPa·s to about 15 mPa·s when diluted with water by a factor $1.1 \times$, $1.2 \times$, $1.3 \times$, $1.4 \times$, $1.5 \times$, $2 \times$, $2.5 \times$, $3 \times$, $4 \times$, $5 \times$ or more.

In preferred embodiments, the thickening agent is present in the first container or vial, or alternatively in a second container or vial, leading to a viscosity of at least $3 \pm 0.5$ mPa·s, preferably at least $4 \pm 0.5$ mPa·s, $5 \pm 0.5$ mPa·s, $6 \pm 0.5$ mPa·s, $7 \pm 0.5$ mPa·s, $8 \pm 0.5$ mPa·s, $9 \pm 0.5$ mPa·s, $10 \pm 0.5$ mPa·s, $11 \pm 0.5$ mPa·s, $12 \pm 0.5$ mPa·s, $13 \pm 0.5$ mPa·s, $14 \pm 0.5$ mPa·s, $15 \pm 0.5$ mPa·s or more.

In some embodiments, each particle in the population of particles comprises a unique identifier or clonal copies thereof, such as a nucleic acid barcode, as defined above.

In some embodiments, the kit may further comprise a container or a vial comprising a gellable solution, as defined above; and optionally at least one density-matching agent, as defined above. Alternatively, the kit may further comprise a container or a vial comprising a gellable solution, as defined above, and another container or vial comprising a density-matching agent, as defined above.

In some embodiments, the kit may further comprise additional containers or vials comprising reagents and solutions for biochemistry and molecular biology assays. Some examples of such reagents and solutions are described in the "Materials" section of Example 5.

In some embodiments, the additional containers or vials may comprise one or several of the following:

- a biological unit wash buffer; it is in particular desirable that the biological unit wash buffer be an isotonic buffer in order to avoid unwanted lysis of the biological units; it is also desirable that the biological unit wash buffer do not contain any nutrient in order to avoid any unwanted metabolic activity of the biological units;
- a lysis buffer; the lysis buffer may comprise one or several salts, pH buffers and chelating agents, as well as one or several of a detergent, a chaotropic agent, an alkaline agent, a solvent, or an enzyme; it is desirable that the detergent, chaotropic agent, alkaline agent, solvent or enzyme be small enough to diffuse through the 3-D network of a hydrogel matrix, *e.g.*, that their molar mass be equal to or smaller than about 1 000 g/mol;
- a hydrogel depolymerization buffer; the hydrogel depolymerization buffer may comprise one or several of a chaotropic agent, a salt, a reducing agent, or an enzyme.
- a particle wash buffer;
- a reverse transcriptase (or RNA-directed DNA polymerase), as defined above;
- a reverse transcription buffer; the reverse transcription buffer may in particular comprise one or several salts and pH buffers, as well as a mix of the 4 deoxyribonucleotide triphosphate (dNTP); it may further comprise template switch oligos (TSO); it my further comprise a ribonuclease (RNAse) inhibitor;
- a DNA polymerase, as defined above;
- a PCR buffer; the PCR buffer may in particular comprise one or several salts and pH buffers, as well as a mix of the 4 deoxyribonucleotide triphosphate (dNTP); it may further comprise SMART™ PCR primers, *e.g.*, with SEQ ID NO: 1;
- an exonuclease;
- a SDS buffer;
- a Tween-20 buffer;
- library preparation primers;
- sequencing primers.

In some preferred embodiments, the kit further comprises at least a hydrogel depolymerization buffer. In some preferred embodiments, the kit further comprises at least a lysis buffer. In some preferred embodiments, the kit further comprises at least a reverse transcriptase and its reverse transcription buffer.

In some embodiments, the kit may further comprise a gelation device comprising a cap, a tube and a piston, as described in European patent application EP 22 305 071.7, the content of which is incorporated herein by reference.

## BRIEF DESCRIPTION OF THE DRAWINGS

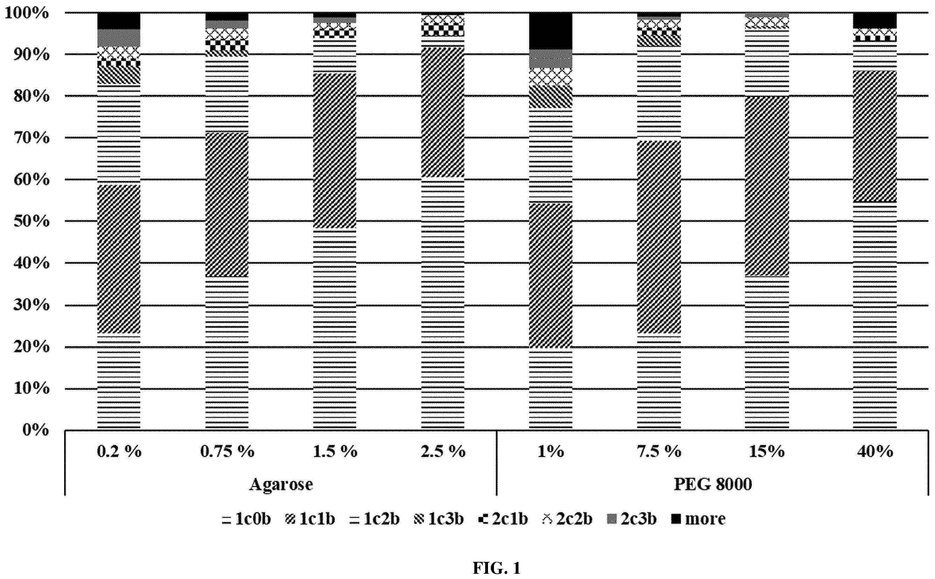

**Figure 1** is a histogram showing the proportion of cells in each kind of complex formed in two thickening agents: SeaPrep™ agarose at 37°C [agarose] and PEG 8000 at 25°C [PEG 8000], with 4 different concentrations for each agent (0.2 %, 0.75 %, 1.5 % and 2.5 % for agarose; 1 %, 7.5 %, 15 % and 40 % for PEG 8000). 1c0b: uncomplexed cells; 1c1b: complex comprising 1 cell-1 bead; 1c2b: complex comprising 1 cell-2 beads; 1c3b: complex comprising 1 cell-3 beads; 2c1b: complex comprising 2 cells-1 bead; 2c2b: complex comprising 2 cells-2 beads; 2c3b: complex comprising 2 cells-3 beads; more: higher order complexes.

**Figures 2A-B** are two graphs showing the viscosity (in mPa·s) of agarose (**Fig. 2A**) and of PEG 8000 (**Fig. 2B**) at various concentrations.

**Figure 3** is a set of photographs showing the incorporation of FSL-biotin in HEK cells, and the presence of streptavidin-AlexaFluor™ 633 conjugates. Upper row: bright field; lower row: fluorescence imaging. Left column: negative control (HEK cells alone); middle column: negative control (HEK cell + streptavidin-AlexaFluor™ 633); right column: HEK cell + FSL-biotin + streptavidin-AlexaFluor™ 633.

**Figure 4** is a schematic workflow of a protocol for single-cell analysis implementing the methods described herein.

**Figure 5** is a schematic representation of a gelation device comprising a cap, a tube and a piston, as described in European patent application EP 22 305 071.7.

## EXAMPLES

The present invention is further illustrated by the following examples.

## Example 1

We aimed at determining whether modulating the final viscosity of suspension of cells and particles had an impact on the formation of cell-particle complexes.

### *Materials and Methods*

We contacted a mix of dissociated HEK293 and NIH/3T3 cells with a solution of cholesteryl-poly(dA) (comprising a 39-dA sequence), to obtain cells exposing on their cell surface poly(dA) nucleic acids.

These poly(dA)-coated cells (100 µL at ≈ 50-100 cells/µL) were then contacted with a solution comprising a thickening agent at various concentrations and 30 µm-barcode beads bearing poly(dT) nucleic acids on their surface (comprising a 30-dT sequence) (100 µL at ≈ 50-100 beads/µL). Two thickening agents were tested: SeaPrep™ agarose at 37°C and PEG 8000 at 25°C, with 4 different concentrations for each agent (0.2 %, 0.75 %, 1.5 % and 2.5 % for agarose; 1 %, 7.5 %, 15 % and 40 % for PEG 8000).

We analyzed the cell-particle complexes by manual counting under a microscope, after sedimentation on a disposable plastic hemocytometer (C-Chip™ or Kova® glasstic® slide).

Finally, we determined the viscosity of each thickening agent at different concentrations. For agarose, measures were carried out on 0.2 %, 0.75 %, 1 %, 1.5 % and 2.5 % samples. The samples were heated for 5 minutes to 60°C, then allowed to cool down to the desired temperature (37°C). A stress-controlled rotational Anton Paar MCR 301 rheometer with a CC10 concentric cylinder was used. The flow curves were determined at equilibrium from 1000 $s^{-1}$ to 1 $s^{-1}$ with 10 points per decade. For PEG 8000, the viscosity was determined by Wisniewska *et al.* (2014. Polymer. 55(18):4651-4657) using an Anton Paar rotational viscometer with a cone-plate at 298 K (25°C). The viscosity for 9 different concentrations of PEG 8000 is given in Table 2 of the supplementary data of Wisniewska *et al.,* which Table is incorporated herein by reference.

### Results

Results are presented in **Figure 1**.

As can be seen on this histogram, a low concentration of thickening agent (*e.g.,* $\leq 0.2$ % agarose or $\leq 1$ % PEG 8000) yielded complexes comprising 2 or more cells bound to 1 or more beads ("2clb", "2c2b", "2c3b", "more"). These complexes are undesirable in single-cell applications, since they represent background noise which distort the final results, with two or more cells indexed with a same barcode and processed as a single cell.

Conversely, a high concentration of thickening agent (*e.g.,* $\geq 2.5$ % agarose or $\geq 40$ % PEG 8000) yielded a majority ($\geq 50$%) of cells which were uncomplexed with barcode beads ("1c0b"). These uncomplexed cells are also undesirable in single-cell applications since they represent a loss of material, which can be scarce in some application.

We estimated thus that the best compromise to obtain less than 50% of material loss and at the same time, less than 10 % of complexes comprising 2 or more cells bound to 1 or more beads, was achieved with 0.75 % or 1.5 % agarose, or with 7.5 % or 15 % PEG 8000.

We finally determined the final viscosity in the cell-particle suspensions.

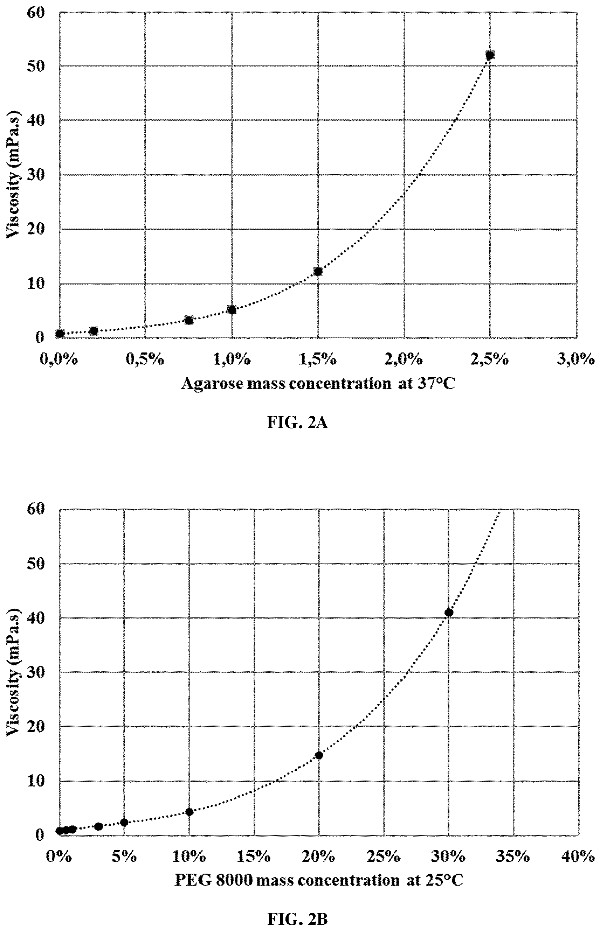

**Figures 2A and 2B** show the viscosity of agarose and PEG 8000, respectively, at the tested concentrations. In doing so, we were able to conclude that the optimal final viscosity of the cell-particle suspension should range from about 3 mPa·s to about 15 mPa·s.

It should be mentioned that temperature is not a relevant factor to be taken into account, except for cell viability. For example, in the case of micron-sized spherical barcode particles and biological units, the surface of contact between these two spheres, and subsequently their probability to adhere to each other, is only dependent of the dimensionless Sommerfeld number s:

$$s = \eta\, v\, R_{eq} \Big/ F_N$$

wherein

$\eta$ is the fluid viscosity,

$v$ is the relative object speed,

$F_N$ is the normal load in the contact, and

$$R_{eq}^{-1} = R_{BU}^{-1} + R_{BC}^{-1}$$, with

$R_{Bu}$ being the radius of the biological unit and

$R_{BC}$ being the radius of the barcode particle.

This formula shows that the temperature does not play any direct role in the complexing contact formation.

### Example 2

#### Materials and methods

In an alternative setup, we first contacted barcode beads bearing poly(dT) nucleic acids on their surface in a solution comprising a thickening agent with cholesteryl-poly(dA), to form barcode beads-poly(dT)···poly(dA)-cholesteryl complexes.

We then contacted these barcode beads-poly(dT)···poly(dA)-cholesteryl complexes with a mix of dissociated HEK293 and NIH/3T3 cells. We analyzed the cell-particle complexes obtained.

### Results

Our data showed that, although barcode beads-poly(dT)···poly(dA)-cholesteryl complexes were formed, the cholesterol moiety did not efficiently anchor into the cell membranes of the dissociated HEK293 and NIH/3T3 cells and only a minority of cell-bead complexes were formed (81.7 % of uncomplexed cells, 14.5 % of cells complexed with beads in a 1:1 ratio; and 3.8 % of higher order complexes).

These observations led to the conclusion that cells should first be coated with nucleic acid molecules (in this case, poly(dA) nucleic acids) before being contacted with beads bearing a complementary nucleic acid sequence (in this case, poly(dT) nucleic acids), as described in Example 1.

### Example 3

HEK293 and NIH/3T3 cells have a diameter typically ranging from ≈ 11 to 18 μm. We wanted to determine whether the cell diameter had any influence on the results obtained in Example 1 above.

#### *Materials and Methods*

The same protocol as in Example 1 was carried out using peripheral blood mononuclear cells (PBMC), which diameter is estimated around 6-8 μm.
Briefly, dissociated PBMC were contacted with a solution of cholesteryl-poly(dA), to obtain cells exposing on their cell surface poly(dA) nucleic acids. These poly(dA)-coated cells (70 μL at ≈ 100 cells/μL) were then contacted with a solution comprising 10 % w/v of PEG 8000 and barcode beads bearing poly(dT) nucleic acids on their surface (70 μL at ≈ 1000 beads/μL). We finally analyzed the cell-particle complexes obtained.

#### *Results*

Only around 32 % of cells were uncomplexed with barcode beads, and 1 % of cells only were found in complexes comprising 2 or more cells bound to 1 or more beads. 51 % of cells were found in 1 cell/1 bead complexes (data not shown).
These results confirm that the same protocol as described in Example 1 can be implemented regardless of the cells' diameter.

### Example 4

The data of Examples 1 and 3 above were obtained using an indirect coupling means through complementary nucleic acid molecules (a membrane-binding moiety (in this case, cholesterol) fused to a nucleic acid molecule (poly(dA)), and barcode beads bearing poly(dT) nucleic acids on their surface).
Binding of cells with barcode beads was thus achieved through the interaction of two complementary nucleic acid molecules, one exposed at the surface of the cells through anchorage of cholesterol molecules in the cell membrane, and the other exposed at the surface of the barcode beads.
We wanted to determine whether a more classical means could be used to trigger this cell-bead interaction.

#### *Materials and Methods*

#### *Cell treatment with FSL-biotin*

FSL-biotin was purchased from Sigma-Aldrich (Ref. F9182). FSL is a technology developed by Kode Biotech, designed to label living cells with functional molecules. FSL molecules are based on three features: a functional component (F), a spacer (S) and a diacyl lipid (L). Thanks to their lipid parts, FSL are able to incorporate into cell membranes. Here, we used FSL with a biotin as functional component in order to add biotin to cells membranes.
Dissociated cells were contacted with FSL-biotin and incubated for 30-60 minutes at 37°C in a cell incubator.
After washing with Gibco™ DPBS (Fisher Scientific, Ref. 11590476), the cell pellet was resuspended in a streptavidin-revealing solution comprising 5 μM of streptavidin-AlexaFluor™ 633 conjugate (ThermoFisher Scientific, Ref. S21375) in DPBS, and incubated for 30 minutes at 37°C in a cell incubator.
Cells were once again washed with Gibco™ DPBS, then transferred to a microplate for visualization under an inverted Axio Observer Apotome microscope (Zeiss).

#### *Biotin-Streptavidin coupling between cells and beads*

In order to test cell-bead coupling through biotin-streptavidin interactions, we used the FSL-biotin-treated HEK cells prepared as described above, and 30 μm streptavidin-functionalized barcode beads.
The cell-bead coupling was performed in a 96-well microplate, where cells had been seeded for 3 hours. 10 000 streptavidin-functionalized barcode beads in 1.5 % agarose and the microplate was incubated at 37°C for 30 minutes.
The mix was then transferred to a microplate for visualization under microscope.

*Results*

*Cell treatment with FSL-biotin*

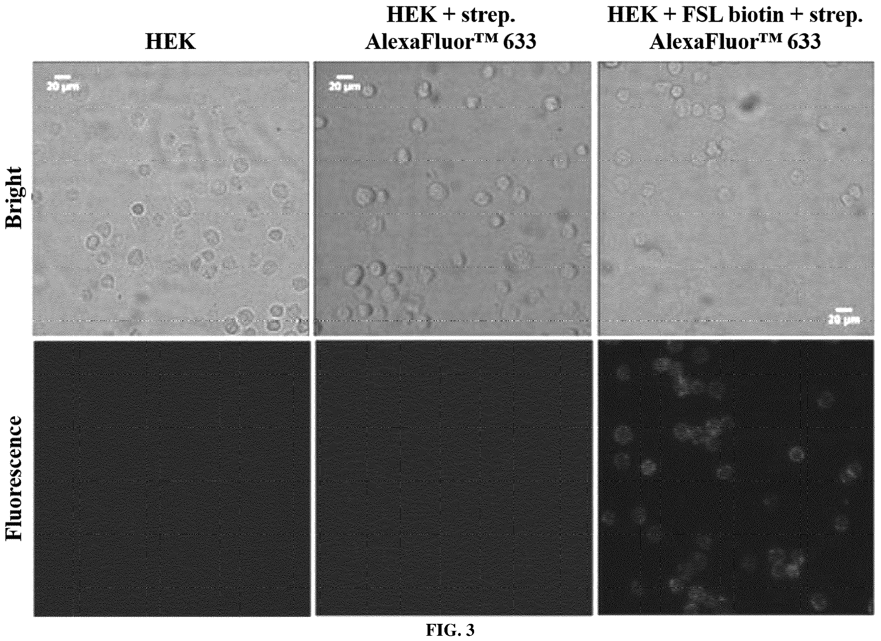

**Figure 3** shows than after FSL-biotin incorporation, and in presence of streptavidin-AlexaFluor™ 633 conjugate, cells are fluorescent. In absence of FSL-biotin but with streptavidin-AlexaFluor™ 633 conjugate (negative control), no fluorescence was observed.

These results thus confirm biotin incorporation in FSL-treated cells.

*Biotin-streptavidin coupling between cells and beads*

Biotin-labeled cell/streptavidin-functionalized barcode bead interactions only occurred with a low frequency (data not shown).

Moreover, the few interactions observed under microscope showed that interactions not only occurred with biotin-labeled cells, but also in a non-specific manner with non-labelled cells (data not shown).

**Conclusion**

Our observations confirmed that, although cells can be functionalized with biotin using a membrane-binding moiety such as a lipid anchoring in the cell membrane, the interaction between these biotin-labeled cells and streptavidin-functionalized barcode beads was not efficient.

Conversely, as demonstrated in Examples 1 and 3, the use of two complementary nucleic acids (such as poly(dA)/poly(dT) sequences) was much more effective for complexing cells and barcode beads.

The cell surface can moreover be functionalized with nucleic acids using a variety of methods, not limited to the use of a lipid moiety anchoring in the membrane. It is conceivable in particular to functionalize cells in a more specific manner, through the use, *e.g.,* of a nucleic acid-antibody fusion molecule or nucleic acid-ligand fusion molecule, where the antibody would target a specific molecule or the ligand would target a specific receptor. Using this alternative strategy, only specific subsets of cells in a cell sample could be targeted and complexed with barcode beads for further single-cell analysis.

This coupling strategy is also readily applicable to any biological unit comprising a membrane: beyond cells, it is conceivable to complex beads or any other kind of particle with nuclei, mitochondria, plastids, endoplasmic reticulum, Golgi apparatus, vacuoles, vesicles, etc.

**Example 5**

The formation of cell-particle complexes may be particularly suitable for single-cell analysis, where the particles can be, *e.g.,* barcode particles such as barcode beads, *i.e.,* particles each bearing clonal copies of a unique identifier.

A detailed protocol for single-cell analysis using such cell-particle complexes is presented hereafter. It is based on the single-cell RNA-seq benchtop kit "ASTERIA©" developed by Scipio Bioscience.

**Materials**

LABELLING AGENT: cholesteryl-poly(dA)

CELL WASH BUFFER

CAPTURE BEADS: barcode beads, polyethylene glycol

DILUTION REAGENT: density-matching agent, gellable solution, nuclease-free water

LYSIS BUFFER: salts, buffer, chelating agent, detergent, nuclease-free water

DEGELATION BUFFER: salts, buffer, chaotropic agent, nuclease-free water

BEADS WASH BUFFER: salts, buffer, nuclease-free water

REVERSE TRANSCRIPTASE

RT SUPERMIX: buffer, dATP, dTTP, dGTP, dCTP, template switch oligonucleotides, RNase inhibitor, nuclease-free water

EXONUCLEASE I

S3 SUPERMIX: buffer, dATP, dTTP, dGTP, dCTP, dN-Smart Randomer (dN-SMRT) oligonucleotides with SEQ ID NO: 1 (AAGCAGTGGTATCAACGCAGAGTGANNNGGNNNB), nuclease-free water

S3 ENZYME: DNA polymerase

TE-SDS BUFFER: Tris/EDTA/SDS, nuclease-free water

TE-TW BUFFER: Tris/EDTA/Tween-20, nuclease-free water

PCR SUPERMIX: buffer, DNA polymerase, dATP, dTTP, dGTP, dCTP, primers, nuclease-free water

LIBRARY PREP PRIMER

READ1 CUSTOM PRIMER

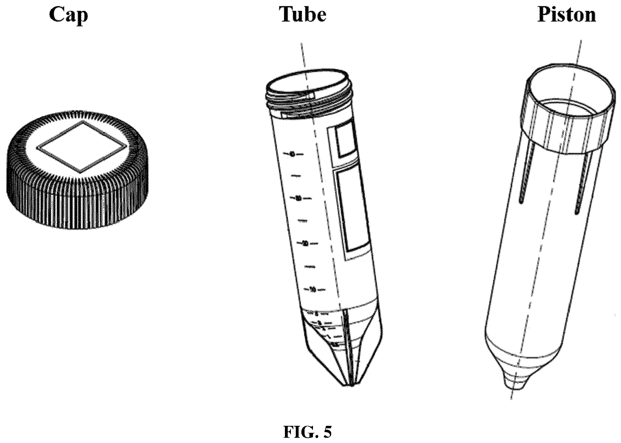

GELATION DEVICE: a TUBE with a first open extremity, a general cylindrical shape, and a closed second extremity with a general convex support area; a PISTON removably insertable inside the TUBE; a CAP cooperating with the first open extremity of the TUBE (**Figure 5**).

*Overview*

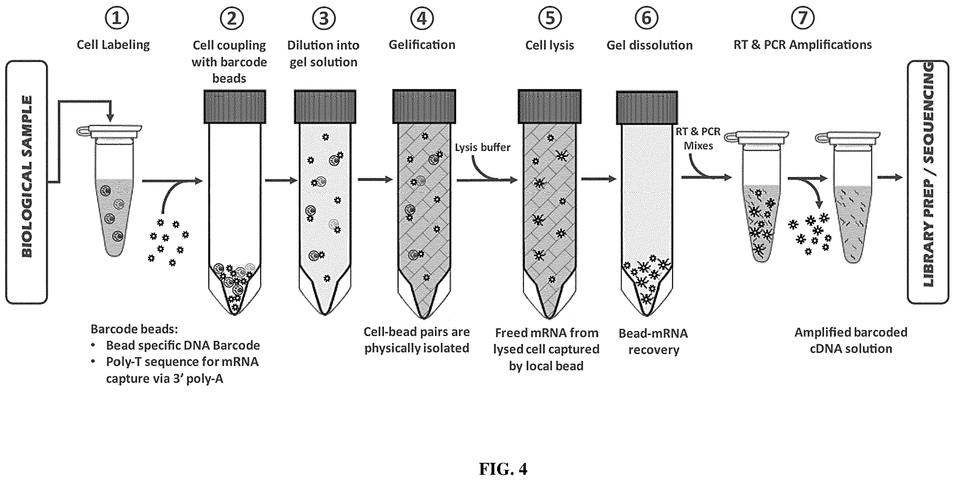

An overview of the proposed protocol is given in **Figure 4.**

Single-cell RNA sample preparation starts from a suspension of dissociated cells.

① Cells are first labelled using LABELLING AGENT to allow interaction with capture beads.

② Mixing labelled cells with CAPTURE BEADS generates cell-bead pairs.

③ The suspension of cell-bead pairs is then diluted into DILUTION REAGENT and spread along the walls of a device to form a thin hydrogel solution layer.

④ Upon incubation on ice, gelation occurs and immobilizes cell-bead pairs.

⑤ Following cell lysis by the addition of LYSIS BUFFER, 3'-poly(A) RNAs hybridize on the 3'-polydT extremities of the barcode beads.

⑥ RNA-loaded barcode beads are then recovered following degelation with DEGELATION BUFFER and transferred into 1.5 mL microtubes.

⑦ Subsequent enzymatic steps include reverse transcription, exonuclease I treatment, second strand synthesis, and PCR to generate amplified barcoded cDNA molecules.

The resulting cDNA library can then be purified before proceeding to library preparation using, *e.g.,* the NEBNext® Ultra II FS DNA Library Prep kit, followed by sequencing, *e.g.*, on an Illumina sequencer.

**Detailed protocol**

*Cell labelling*

Transfer approximately 100 000 freshly harvested and dissociated cells into a 1.5-mL microtube.

Pellet your cells by centrifuging at appropriate speed and duration (*e.g.,* ≈ 200 xg for large cells such as cardiomyocytes, ≈ 300 xg for medium size cells such as NIH or HEK cells, or ≈ 500 xg for small cells such as PBMC). Carefully remove supernatant leaving no more than 20 μL.

Prepare LABELLING MIX by mixing 25 μL/sample of CELL WASH BUFFER and 2.8 μL/sample of LABELLING AGENT. Add 25 μL of LABELLING MIX to the cell pellet; gently pipette up/down a few times to resuspend the cells; incubate for 5 minutes at 37°C.

Add 250 μL of cold CELL WASH BUFFER to your labelled cells; centrifuge cells at appropriate speed and duration; carefully remove 250 μL of supernatant.

Add 250 μL of cold CELL WASH BUFFER; gently pipet up/down a few times to resuspend the cells; centrifuge cells at appropriate speed and duration; carefully remove 250 μL of supernatant.

Add 100 μL of cold CELL WASH BUFFER; resuspend cells.

Count labelled cells.

In a new 1.5-mL tube, prepare 120 μL of a labelled cell suspension at a concentration of 100 cells/μL using CELL WASH BUFFER.

Keep on ice until coupling, preferably for at most 30 minutes.

*Cell Coupling & Gelation*

Briefly spin down the CAPTURE BEADS; thoroughly homogenize CAPTURE BEADS suspension by gentle pipetting; transfer 100 μL of the CAPTURE BEADS into the GELATION DEVICE tube.

Homogenize labelled cells suspension by gentle pipetting; thoroughly homogenize the CAPTURE BEADS suspension by gentle pipetting; proceed immediately to next step to prevent bead sedimentation.

Transfer 100 μL of the labelled cell suspension (10 000 cells) into the middle height of the CAPTURE BEADS suspension.

Immediately perform 5 up and down cycles of pipetting with the pipette tip positioned from about 3 mm to about 5 mm from the bottom of the GELATION DEVICE tube, moving at 90 bpm.

Add 2 mL of DILUTION REAGENT at the bottom of the GELATION DEVICE tube.

Close the GELATION DEVICE tube with the GELATION DEVICE cap and homogenize by rotating the tube 15 times at approximately 3 rotations per second with an angle of 30°.

Remove the GELATION DEVICE cap and set the tube vertically. Slowly insert the GELATION DEVICE piston in the GELATION DEVICE tube while keeping both vertical.

Wait for 1 minute with the GELATION DEVICE piston inserted; place the assembled GELATION DEVICE (tube + piston) vertically in ice and incubate for 20 minutes to allow for complete gelation.

*Cell Lysis*

Slowly pull out the GELATION DEVICE piston from the GELATION DEVICE tube; the gel stays inside the tube and the piston should come out clean.

Homogeneize LYSIS BUFFER; add 7.5 mL of LYSIS BUFFER to the sample; close the GELATION DEVICE tube with the GELATION DEVICE cap; seal the GELATION DEVICE cap with parafilm to prevent any leakage.

Put the GELATION DEVICE tube on an orbital shaker at around 60 rpm; keep on agitation for 60 minutes at room temperature.

*Degelation*

Following lysis incubation, proceed immediately to degelation without putting the sample on ice.

Vortex the DEGELATION BUFFER for 15 seconds; add 3.75 mL of DEGELATION BUFFER in the GELATION DEVICE tube; close the GELATION DEVICE tube with the GELATION DEVICE cap; seal the GELATION DEVICE cap with parafilm to prevent any leakage.

Vortex the GELATION DEVICE tube for 5 minutes at maximum speed, or until no gel fragments are visible.

Centrifuge the GELATION DEVICE tube for 3 minutes at 1 000 xg in a swinging bucket centrifuge to pellet the CAPTURE BEADS; remove supernatant carefully; leave around 500 μL.

Add 5 mL ice-cold BEADS WASH BUFFER directly on the bead pellet; centrifuge the GELATION DEVICE tube for 3 minutes at 1 000 xg in a swinging bucket centrifuge to pellet the CAPTURE BEADS; remove supernatant carefully; leave around 500 μL.

Transfer the CAPTURE BEADS suspension into a DNase/RNase-free 1.5 mL microtube; spin for 60 seconds on a mini-centrifuge; remove supernatant carefully.

Add 250 μL ice-cold BEADS WASH BUFFER; spin for 30 seconds on a mini-centrifuge; remove supernatant carefully. Repeat twice.

Adjust the final volume of CAPTURE BEADS suspension to 20 μL ± 1 μL using BEADS WASH BUFFER.

*Reverse transcription*

Spin and homogenize RT SUPERMIX; add 75 μL of RT SUPERMIX in the 1.5 mL microtube containing the 20 μL of CAPTURE BEADS suspension.
Add 5 μL of REVERSE TRANSCRIPTASE; homogenize well by pipetting.
Transfer the sample into a heat block and incubate as follows: 10 minutes at 25°C, then 90 minutes at 42°C. Preferably, perform enzymatic reaction incubation with agitation (1 250 rpm) and heated lid.
Place the sample tube on ice for 2 minutes and then spin briefly.

*Exonuclease I treatment*

Add 5 μL of EXONUCLEASE I; homogenize samples by pipetting.
Incubate samples for 50 minutes at 37°C.
Spin samples for 20 seconds on benchtop centrifuge; carefully remove 80 μL of supernatant.
Add 250 μL of TE-SDS BUFFER; spin samples for 20 seconds on a benchtop centrifuge; carefully remove 250 μL of supernatant.

*Denaturation*

Add 100 μL of 0.1 M NaOH solution; spin the sample for 20 seconds on a benchtop centrifuge; remove 100 μL of supernatant.
Add 50 μL of 0.1 M NaOH solution; rotate the sample for 5 minutes on a microtube rotator.
Spin the sample for 20 seconds on a benchtop centrifuge; remove 50 μL of supernatant.
Add 250 μL of TE-TW BUFFER; spin the sample for 20 seconds on a benchtop centrifuge; remove 200 μL of supernatant.
Add 250 μL of BEADS WASH BUFFER; spin the sample for 20 seconds on a benchtop centrifuge; remove 250 μL of supernatant.
Adjust the residual volume to 20 μL using BEADS WASH BUFFER.

*Second Strand Synthesis*

Incubate the S3 SUPERMIX for 5 minutes at 70°C; immediately incubate on ice for 1 minute; spin briefly and homogenize by pipetting.
Add 77.5 μL of S3 SUPERMIX and 2.5 μL of S3 ENZYME to sample tube; thoroughly homogenize the sample by pipetting.
Incubate for 1 hour at 37°C. Preferably, perform enzymatic reaction incubation with agitation (1 250 rpm) and heated lid.
Spin the sample for 20 seconds on a benchtop centrifuge; remove 60 μL of supernatant.
Add 250 μL of TE-TW BUFFER; spin the sample for 20 seconds on a benchtop centrifuge; carefully remove 250 μL of supernatant. Repeat twice.
Add 250 μL of nuclease-free water; spin the sample for 20 seconds on a benchtop centrifuge; carefully remove 140 μL of supernatant; adjust the residual volume to 160 μL with nuclease-free water.

*Polymerase Chain Reaction*

Add 240 μL of PCR SUPERMIX to the sample; homogenize beads by pipetting and split into PCR tubes, with 50 μL in each.
Close tubes tightly; transfer to thermocycler.
Run the PCR program with the following parameters:

| Step | | Cycles | Temperature | Duration |
|---|---|---|---|---|
| **1** | Initial denaturation | --- | 95°C | 3 minutes |

(continued)

|   | Step | Cycles | Temperature | Duration |
|---|---|---|---|---|
| **2** | Denaturation | 4 | 98°C | 20 seconds |
|   | Annealing |   | 65°C | 45 seconds |
|   | Extension |   | 72°C | 3 minutes |
| **3** | Denaturation | 9 | 98°C | 20 seconds |
|   | Annealing |   | 67°C | 20 seconds |
|   | Extension |   | 72°C | 3 minutes |
| **4** | Final extension | --- | 72°C | 5 minutes |
| **5** | Store | --- | 4°C | ∞ |

*Sample purification using SPRIselect*

Adapted from the SPRIselect user guide (Beckman Coulter).

Spin PCR strip tubes; transfer 45 μL of CAPTURE BEADS-free supernatant from each of the PCR reaction tubes into a single 1.5-mL microtube.

Add 216 μL of SPRIselect beads (Beckman Coulter) (0.6 x of PCR reaction volume) to the sample tube; mix the total reaction volume by pipetting 10 times and incubate at room temperature for 1 minute.

Place the 1.5-mL microtube on an appropriate magnetic stand and allow the SPRIselect beads to settle to the magnet; remove and discard the cleared supernatant.

Add 1 mL of 85 % ethanol to each tube and incubate at room temperature for 30 seconds; remove and discard the ethanol supernatant. Repeat twice.

Ensure no residual ethanol droplets remain; dry the magnetic beads at room temperature for 3 minutes maximum; remove the microtube from the magnet.

Add 100 μL of nuclease-free water to the microtube and resuspend SPRIselect beads by pipetting 20 times; incubate at room temperature for 1 minute.

Place the microtube on an appropriate magnetic stand or plate and allow the SPRIselect beads to settle to the magnet; transfer the cleared solution to an appropriate storage tube, being careful not to aspirate any magnetic SPRIselect beads.

*Library Preparation*

Sample 5 ng of SPRIselect-purified cDNA amplicons and proceed according to *"Protocol for FS DNA Library Prep Kit (E7805, E6177) with Inputs ≤100 ng"* (New England Biolabs, accessed online on April 06, 2022 at https://international.neb.com/protocols/2017/1 0/25/protocol- for-fs-dna -library-prep-kite7805-e6177-with-inputs-less-than-or-equal-to-100-ng; the content of which is incorporated herein by reference), with the following adjustments:

- in §1.1 of the above-protocol: target fragmentation size of 300-700 bp and 10 minutes of incubation;
- in §1.4.1A of the above-protocol: replace Universal PCR Primer/i5 Primer with LIBRARY PREP PRIMER.

*Sequencing*

The sample is then sequenced on Illumina sequencing platform according to manufacturer specification in order to obtain 30 bases on read 1 (barcode side) and 70 bases on read 2 (cDNA side).

*Data analysis*

The analysis of the sequencing data is performed using Cytonaut©. Cytonaut© is a software-as-a-service application provided by Scipio bioscience for the analysis of single-cell RNA sequencing data of samples prepared with Scipio bioscience ASTERIA© kit, using the following website: https://www.cytonaut-scipio.bio.

Alternatively, the analysis of the sequencing data can be performed using reference genome sequences and annotations (such as Ensembl release v99 for human species (GRCh38) and mouse species (GRCm38)) and reliable and commonly used open-source tools (UMI-tools, STAR, FastQC, RSeqQC, Picard, Samtools, FeatureCounts, etc.)

to successively performs read quality control, extraction, alignment, deduplication and assignment.

**Claims**

1. A method of complexing biological units with particles, comprising contacting a population of dissociated biological units with a solution comprising (i) a population of particles and (ii) at least one thickening agent, in the presence of at least one means for binding a biological unit, to obtain a suspension of biological unit-particle complexes, wherein the final viscosity of the suspension of biological unit-particle complexes ranges from about 3 mPa·s to about 15 mPa·s.

2. The method according to claim **1,** wherein the at least one means for binding a biological unit comprises a group or moiety selected from the group consisting of nucleic acids, lipids, proteins or fragments thereof, peptides, antibodies or fragments thereof, carbohydrates, vitamins or derivatives thereof, coenzymes or derivatives thereof, receptor ligands or derivatives thereof, and hydrophobic groups.

3. The method according to claim **1** or **2,** wherein the at least one means for binding a biological unit comprises at least a first nucleic acid molecule fused to a biological unit-binding moiety and at least a second nucleic acid molecule,

   wherein the at least second nucleic acid molecule is bound or otherwise attached to the particles of the population of particles,
   wherein the at least first nucleic acid molecule and the at least second nucleic acid molecule are capable of hybridization.

4. The method according to any one of claims **1** to **3,** comprising the following steps in the recited order:

   a) contacting the population of dissociated biological units with a biological unit-binding moiety fused to at least a first nucleic acid molecule, and
   b) contacting the population of dissociated biological units from a) with the population of particles, wherein at least a second nucleic acid molecule is bound or otherwise attached to the particles of the population of particles;

   wherein the at least first nucleic acid molecule and the at least second nucleic acid molecule are capable of hybridization.

5. The method according to claim **3** or **4,** wherein the biological unit-binding moiety is selected from the group consisting of lipids, antibodies or fragments thereof, and receptor ligands.

6. The method according to claim **5,** wherein the lipid is selected from the group consisting of sterol lipids, fatty acids, glycerolipids, glycerophospholipids, sphingolipids, saccharolipids, polyketides and prenol lipids; preferably wherein the lipid is cholesterol.

7. The method according to any one of claims **4** to **6,** wherein the at least first nucleic acid molecule comprises or consists of a poly(dA) sequence, and the at least second nucleic acid molecule comprises or consists of a poly(dT) or poly(U) sequence.

8. The method according to any one of claims **1** to **7,** wherein the biological units are biological structures comprising a lipid membrane, preferably selected from the group consisting of cells, nuclei, mitochondria, plastids, endoplasmic reticulum, Golgi apparatus, vacuoles, vesicles, organoids, spheroids, and cell tissues.

9. The method according to any one of claims **1** to **8,** wherein the particles are beads.

10. The method according to any one of claims **1** to **9,** wherein each particle in the population of particles comprises a unique identifier or clonal copies thereof, preferably the identifier is a nucleic acid barcode.

11. The method according to any one of claims **1** to **10,** wherein the suspension of biological unit-particle complexes comprises:

    - less than 50 % of uncomplexed biological units, and/or

- less than 10 % of biological units complexed with a particle concurrently with one or several other biological units.

12. A method of trapping discrete biological unit-particle complexes in a hydrogel, said method comprising the following steps:

   a) complexing biological units with particles to form a suspension of biological unit-particle complexes according to the method of any one of claims **1** to **11,** wherein the suspension of biological unit-particle complexes comprises, or is diluted with, a gellable solution optionally comprising at least one density-matching agent,
   b) forming a hydrogel matrix trapping the biological unit-particle complexes, preferably in a discrete fashion.

13. A method of uniquely identifying a biological unit's analytes, said method comprising the following steps:

   a) complexing biological units with particles to form a suspension of biological unit-particle complexes according to the method of any one of claims **1** to **11,** wherein each particle comprises a unique identifier or clonal copies thereof, preferably the unique identifier is a nucleic acid barcode, wherein the suspension of biological unit-particle complexes comprises, or is diluted with, a gellable solution optionally comprising at least one density-matching agent,
   b) forming a hydrogel matrix trapping the biological unit-particle complexes, preferably in a discrete fashion, and
   c) labelling the biological unit's analytes of each biological unit-particle complex with the unique identifier.

14. The method according to claim **13,** comprising a further step of sequencing the biological unit's analytes labelled with their unique identifier.

15. A kit for implementing the methods according to any one of claims **1** to **14,** comprising:

   - a first container or vial containing at least a first nucleic acid molecule fused to a biological unit-binding moiety;
   - a second container or vial containing a population of particles, said particles comprising at least a second nucleic acid molecule;
   - at least one thickening agent, either in the second container or vial, or in a third container or vial,

   wherein the at least first nucleic acid molecule and the at least second nucleic acid molecule are capable of hybridization.

FIG. 1

FIG. 2A

FIG. 2B

FIG. 3

FIG. 4

Cap Tube Piston

FIG. 5

Europäisches
Patentamt

European
Patent Office

Office européen
des brevets

# EUROPEAN SEARCH REPORT

Application Number

EP 22 30 5661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 417 249 A1 (UNIV CALIFORNIA [US]) 15 February 2012 (2012-02-15) * figure 1 * ----- | 1-15 | INV. A61K47/69 C12N5/00 C12Q1/6806 |
| X | RAVI A. CHANDRA ET AL: "Programmable Cell Adhesion Encoded by DNA Hybridization", ANGEWANDTE CHEMIE INTERNATIONAL EDITION, vol. 45, no. 6, 30 January 2006 (2006-01-30), pages 896-901, XP055063360, ISSN: 1433-7851, DOI: 10.1002/anie.200502421 * figure 1 * ----- | 1-15 | |
| X | BORISENKO GRIGORY G. ET AL: "DNA modification of live cell surface", NUCLEIC ACIDS RESEARCH, vol. 37, no. 4, 1 March 2009 (2009-03-01), pages e28-e28, XP055973505, GB ISSN: 0305-1048, DOI: 10.1093/nar/gkn1034 Retrieved from the Internet: URL:https://www.ncbi.nlm.nih.gov/pmc/artic les/PMC2651772/pdf/gkn1034.pdf> * the whole document * ----- | 1-15 | TECHNICAL FIELDS SEARCHED (IPC) C12N A61K C12Q |
| X | ERIK S. DOUGLAS ET AL: "Self-assembled cellular microarrays patterned using DNA barcodes", LAB ON A CHIP, vol. 7, no. 11, 1 January 2007 (2007-01-01), page 1442, XP055039830, ISSN: 1473-0197, DOI: 10.1039/b708666k * the whole document * ----- -/-- | 1-15 | |

The present search report has been drawn up for all claims

| Place of search | Date of completion of the search | Examiner |
|---|---|---|
| The Hague | 21 October 2022 | Gabriels, Jan |

EPO FORM 1503 03.82 (P04C01)

Europäisches
Patentamt

European
Patent Office

Office européen
des brevets

# EUROPEAN SEARCH REPORT

Application Number

EP 22 30 5661

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | SONNY C. HSIAO ET AL: "Direct Cell Surface Modification with DNA for the Capture of Primary Cells and the Investigation of Myotube Formation on Defined Patterns", LANGMUIR, vol. 25, no. 12, 16 June 2009 (2009-06-16), pages 6985-6991, XP055063345, ISSN: 0743-7463, DOI: 10.1021/la900150n * the whole document * | 1-15 | |
| X | WO 2012/155110 A1 (MASSACHUSETTS INST TECHNOLOGY [US]; BHATIA SANGEETA N [US] ET AL.) 15 November 2012 (2012-11-15) * page 25 - page 26 * | 1-15 | |
| A | FATEMEH MOMEN-HERAVI ET AL: "Impact of Biofluid Viscosity on Size and Sedimentation Efficiency of the Isolated Microvesicles", FRONTIERS IN PHYSIOLOGY, vol. 3, 1 January 2012 (2012-01-01), XP055189135, ISSN: 1664-042X, DOI: 10.3389/fphys.2012.00162 * the whole document * | 1,11 | TECHNICAL FIELDS SEARCHED (IPC) |

The present search report has been drawn up for all claims

| Place of search | Date of completion of the search | Examiner |
|---|---|---|
| The Hague | 21 October 2022 | Gabriels, Jan |

## ANNEX TO THE EUROPEAN SEARCH REPORT
## ON EUROPEAN PATENT APPLICATION NO.

EP 22 30 5661

This annex lists the patent family members relating to the patent documents cited in the above-mentioned European search report.
The members are as contained in the European Patent Office EDP file on
The European Patent Office is in no way liable for these particulars which are merely given for the purpose of information.

21-10-2022

| Patent document cited in search report | | Publication date | Patent family member(s) | | Publication date |
|---|---|---|---|---|---|
| EP 2417249 | A1 | 15-02-2012 | CA | 2757884 A1 | 14-10-2010 |
| | | | CN | 102575228 A | 11-07-2012 |
| | | | CN | 107326002 A | 07-11-2017 |
| | | | CN | 113186149 A | 30-07-2021 |
| | | | EP | 2417249 A1 | 15-02-2012 |
| | | | ES | 2924287 T3 | 05-10-2022 |
| | | | JP | 5964746 B2 | 03-08-2016 |
| | | | JP | 2012523569 A | 04-10-2012 |
| | | | JP | 2016168062 A | 23-09-2016 |
| | | | US | 2012142088 A1 | 07-06-2012 |
| | | | US | 2016319238 A1 | 03-11-2016 |
| | | | WO | 2010118235 A1 | 14-10-2010 |
| WO 2012155110 | A1 | 15-11-2012 | US | 2014212910 A1 | 31-07-2014 |
| | | | US | 2019376024 A1 | 12-12-2019 |
| | | | WO | 2012155110 A1 | 15-11-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018203141 A
- US 20160289669 A
- US 20160265069 A
- US 20160060621 A
- US 20150376609 A
- WO 2016061517 A
- WO 2005003304 A
- WO 2015126766 A
- WO 2016130704 A
- WO 201661517 A
- WO 201595226 A
- WO 2016003814 A
- WO 2015200869 A
- WO 2014124338 A
- WO 2014093676 A
- US 2015066385 A
- WO 2014189957 A
- EP 22305071 A

### Non-patent literature cited in the description

- **MACOSKO et al.** *Cell,* 2015, vol. 161 (5), 1202-1214
- **KLEIN ; MACOSKO.** *Lab Chip.,* 2017, vol. 17 (15), 2540-2541
- **QUAKE.** *Trends Genet,* 2021, vol. 37 (12), 1064-1068
- **CABLE et al.** *Ann N Y Acad Sci.,* 2021, vol. 1506 (1), 74-97
- **MEREU et al.** *Nat Biotechnol.,* 2020, vol. 38 (6), 747-755
- **DAVIS-MARCISAK et al.** *Cancer Cell,* 2021, vol. 39 (8), 1062-1080
- **KORNBERG A. ; BAKER T.A.** DNA replication. W.H. Freeman, 1992, 113-225
- **MACOSKO et al.** *Cell,* 2015, vol. 161, 1202-1214
- **FAN et al.** *Science,* 2015, vol. 347 (6222), 1258367
- **KLEIN et al.** *Cell,* 2015, vol. 161 (5), 1187-201
- **GIERAHN et al.** *Nat Methods.,* 2017, vol. 14 (4), 395-398
- **ZONG et al.** *Science,* 2012, vol. 338 (6114), 1622-6
- **LU et al.** *Science,* 2012, vol. 338 (6114), 1627-30
- **HUTCHISON et al.** *Proc Natl Acad Sci USA.,* 2005, vol. 102 (48), 17332-6
- **LEUNG et al.** *Proc Natl Acad Sci USA.,* 2016, vol. 113 (30), 8484-9
- **WANG et al.** *Cell,* 2012, vol. 150 (2), 402-12
- **MARCY et al.** *PLoS Genet.,* 2007, vol. 3 (9), 1702-8
- **GOLE et al.** *Nat Biotechnol.,* 2013, vol. 31 (12), 1126-32
- **ZHANG et al.** *Nat Biotechnol.,* 2006, vol. 24 (6), 680-6
- **AMINI et al.** *Nat Genet.,* 2014, vol. 46 (12), 1343-9
- **KULESHOV et al.** *Nat Biotechnol.,* 2014, vol. 32 (3), 261-6
- **KAPER et al.** *Proc Natl Acad Sci USA.,* 2013, vol. 110 (14), 5552-7
- **PETERS et al.** *Nature,* 2012, vol. 487 (7406), 190-5
- **ZHENG et al.** *Nat Biotechnol.,* 2016, vol. 34 (3), 303-11
- **BUENROSTRO et al.** *Nature,* 2015, vol. 523 (7561), 486-90
- **BUENROSTRO et al.** *Nat Methods,* 2013, vol. 10 (12), 1213-8
- **CHRISTIANSEN et al.** *Methods Mol Biol.,* 2017, vol. 1551, 207-221
- *Polymer,* 2014, vol. 55 (18), 4651-4657